(19) Europäisches Patentamt
European Patent Office
Office européen des brevets

(11) **EP 4 389 871 A1**

(12) **EUROPEAN PATENT APPLICATION**
published in accordance with Art. 153(4) EPC

(43) Date of publication:
**26.06.2024 Bulletin 2024/26**

(21) Application number: **22858412.4**

(22) Date of filing: **10.08.2022**

(51) International Patent Classification (IPC):
**C12M 1/34** (2006.01)　　　**C12M 1/00** (2006.01)
**C12M 3/00** (2006.01)　　　**C12Q 1/02** (2006.01)
**G01J 9/02** (2006.01)　　　**G01N 21/45** (2006.01)
**G01N 33/483** (2006.01)

(52) Cooperative Patent Classification (CPC):
**C12M 1/00; C12M 1/34; C12M 3/00; C12Q 1/02;
G01J 9/02; G01N 21/45; G01N 33/483**

(86) International application number:
**PCT/JP2022/030569**

(87) International publication number:
**WO 2023/022091 (23.02.2023 Gazette 2023/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.08.2021　JP 2021132304**

(71) Applicant: **NIKON CORPORATION**
**Minato-ku**
**Tokyo 108-6290 (JP)**

(72) Inventors:
• **UEDA Takehiko**
**Tokyo 108-6290 (JP)**

• **SAKAGAMI Junko**
**Tokyo 108-6290 (JP)**
• **KOBAYASHI Ryo**
**Tokyo 108-6290 (JP)**
• **TAKUBO Makiko**
**Tokyo 108-6290 (JP)**
• **ISHIZAWA Naoya**
**Tokyo 108-6290 (JP)**
• **TANAKA Shuhei**
**Tokyo 108-6290 (JP)**

(74) Representative: **Hoffmann Eitle**
**Patent- und Rechtsanwälte PartmbB**
**Arabellastraße 30**
**81925 München (DE)**

(54) **ANALYSIS SYSTEM, OBSERVATION CONTAINER, ANALYSIS METHOD AND PROGRAM**

(57)　An analysis system includes: an acquisition unit configured to acquire a cell image including images of a plurality of cultured cells which are cultured in a medium; and an image analyzing unit configured to analyze living states of the plurality of cultured cells included in the cell image on the basis of luminance values and cell diameters of the cultured cells included in the cell image.

EP 4 389 871 A1

FIG. 1

**Description**

[Technical Field]

An embodiment of the present invention relates to an analysis system, an observation container, an analysis method, and a program.

Priority is claimed on Japanese Patent Application No. 2021-132304, filed August 16, 2021, the content of which is incorporated herein by reference.

[Background Art]

In the related art, a method of determining characteristics of ellipsoidal transparent particles in a transparent medium and counting the ellipsoidal transparent particles is known (for example, see Patent Document 1).

[Citation List]

[Patent Document]

[Patent Document 1]
Japanese Patent No. 6039570

[Summary of Invention]

According to an aspect of the present invention, there is provided an analysis system including: an acquisition unit configured to acquire a cell image including images of a plurality of cultured cells which are cultured in a medium; and an image analyzing unit configured to analyze living states of the plurality of cultured cells included in the cell image on the basis of luminance values and cell diameters of the cultured cells included in the cell image.

According to another aspect of the present invention, there is provided an observation container including an accommodation unit for accommodating a medium and a plurality of cells and used to acquire and observe a quantitative phase image of the plurality of cells, wherein the accommodation unit is formed such that an inner surface shape of the accommodation unit in an optical-axis direction of an observation optical system satisfies conditions in which the cells floating in the medium are made to stay in an in-focus range of the observation optical system for a predetermined observation time.

According to another aspect of the present invention, there is provided an analysis method that is performed by a computer, the analysis method including: a step of acquiring a cell image including images of a plurality of cultured cells; a step of deriving a refractive index difference between a medium in the plurality of cultured cells and the cells included in the cell image acquired in the acquiring step; and a step of deriving one or both of a survival rate of the cultured cells included in the cell image and a living cell density of living cells in the cultured cells on the basis of refractive index difference information identifying the refractive index difference derived in the deriving step.

According to another aspect of the present invention, there is provided an analysis method that is performed by a computer, the analysis method including: a step of acquiring a cell image including images of one or more cultured cells; a step of deriving a refractive index difference between a medium in the one or more cultured cells and the cells included in the cell image acquired in the acquiring step; and a step of determining that a group having a high refractive index difference out of the one or more cultured cells included in the cell image is cells with an excellent antibody production capacity out of the cultured cells on the basis of information identifying the refractive index difference derived in the deriving step.

According to another aspect of the present invention, there is provided a program causing a computer to perform: a step of acquiring a cell image including images of a plurality of cultured cells; a step of deriving a refractive index difference between a medium in the plurality of cultured cells and the cells included in the cell image acquired in the acquiring step; and a step of deriving one or both of a survival rate of the cultured cells included in the cell image and a living cell density of living cells in the cultured cells on the basis of refractive index difference information identifying the refractive index difference derived in the deriving step.

According to another aspect of the present invention, there is provided a program causing a computer to perform: a step of acquiring a cell image including images of one or more cultured cells; a step of deriving a refractive index difference between a medium and a cell in the one or more cultured cells included in the cell image acquired in the acquiring step; and a step of determining that a group having a high refractive index difference out of the one or more cultured cells included in the cell image is cells with an excellent antibody production capacity out of the cultured cells on the basis of information identifying the refractive index difference derived in the deriving step.

[Brief Description of Drawings]

FIG. 1 is a conceptual diagram illustrating a configuration of an analysis system according to an embodiment.
FIG. 2 is a diagram illustrating a configuration of an analysis device included in the analysis system according to the embodiment.
FIG. 3 is a conceptual diagram illustrating change of an intensity distribution of light transmitted by a phase object.
FIG. 4 is a diagram illustrating a method of detecting light from a sample S which is performed to calculate a phase distribution $\phi$.
FIG. 5 is a diagram illustrating arrangement examples of a focal point of an objective lens 21a and measurement planes i1 to i3 when a sample S is sampled.
FIG. 6 is a diagram illustrating a sampling interval.
FIG. 7 is a conceptual diagram illustrating a method of setting a sampling interval $\Delta x$.
FIG. 8A is a diagram illustrating Example 1 of a container.
FIG. 8B is a diagram illustrating Example 2 of a container.
FIG. 9 is a diagram illustrating an example of a bright-field image.
FIG. 10 is a diagram illustrating an example of a quantitative phase image.
FIG. 11 is a diagram illustrating a quantified phase analysis result in the analysis system according to the embodiment.
FIG. 12 is a diagram illustrating a refractive index difference between a medium and a cell.
FIG. 13 is a diagram illustrating a method of determining a binarization threshold value.
FIG. 14 is a flowchart illustrating Example 1 of an operation flow in the analysis system according to the embodiment.
FIG. 15 is a flowchart illustrating Example 2 of an operation flow in the analysis system according to the embodiment.
FIG. 16 is a flowchart illustrating Example 3 of an operation flow in the analysis system according to the embodiment.
FIG. 17 is a conceptual diagram illustrating an example of an analysis result which is displayed by the analysis device included in the analysis system according to the embodiment.
FIG. 18 is a diagram illustrating a configuration of an analysis device included in an analysis system according to a modified example of the embodiment.
FIG. 19 is a diagram illustrating a culturing device.
FIG. 20 is a diagram illustrating an example of a relationship between a specific antibody production rate and an average value of a refractive index difference ($\Delta n$).
FIG. 21 is a flowchart illustrating an example of an operation flow in an analysis system according to a modified example of the embodiment.
FIG. 22 is a flowchart illustrating an example of an operation flow in an analysis system according to a modified example of the embodiment.
FIG. 23 is a conceptual diagram illustrating Example 1 of a display screen which is displayed by the analysis device included in the analysis system according to a modified example of the embodiment.
FIG. 24 is a conceptual diagram illustrating Example 2 of a display screen which is displayed by the analysis device included in the analysis system according to a modified example of the embodiment.
FIG. 25 is a diagram illustrating an example of a bright-field image.
FIG. 26 is a diagram illustrating an example of a relationship between an objective lens NA and a focal depth.
FIG. 27 is a diagram illustrating an example of an observation container.
FIG. 28 is a diagram illustrating an example of an observation container.
FIG. 29A is a diagram illustrating Example 1 of an observation container.
FIG. 29B is a diagram illustrating Example 2 of an observation container.
FIG. 29C is a diagram illustrating Example 3 of an observation container.
FIG. 30A is a diagram illustrating Example 4 of an observation container.
FIG. 30B is a diagram illustrating Example 5 of an observation container.
FIG. 30C is a diagram illustrating Example 6 of an observation container.
FIG. 30D is a diagram illustrating Example 7 of an observation container.
FIG. 31 is a diagram illustrating Example 8 of an observation container.
FIG. 32 is a diagram illustrating Example 9 of an observation container.

[Description of Embodiments]

(Embodiment)

Hereinafter, an analysis system, an analysis method, and a program according to an embodiment will be described with reference to the accompanying drawings.

An analysis system 1 according to an embodiment serves to apply illumination light while disposing a focal point (that is, an in-focus plane) of an objective lens at a plurality of positions separated at intervals Δz in a sample, to detect light from areas at which the focal point is arranged (that is, in-focus planes), and to generate a quantitative phase image on the basis of data acquired through the detection. The interval Δz is set on the basis of a numerical aperture of the objective lens, a wavelength of illumination light, and a refractive index between the objective lens and the sample.

Here, a quantitative phase image is an image representing (obtained by visualizing) a phase which is a product of thickness and change in a refractive index in a sample. An existing phase difference image or an existing differential interference image is an interference image in which amplitude is mixed into phases and thus cannot perfectly quantify a phase of a sample (such an image can be quantified under only conditions in which the amplitude can be ignored and the phase is small), but the quantitative phase image can represent a quantitative phase even when the phase of a sample changes to various values such as two times, three times, or four times and thus can also be referred to as an image obtained by quantifying the phase of the sample.

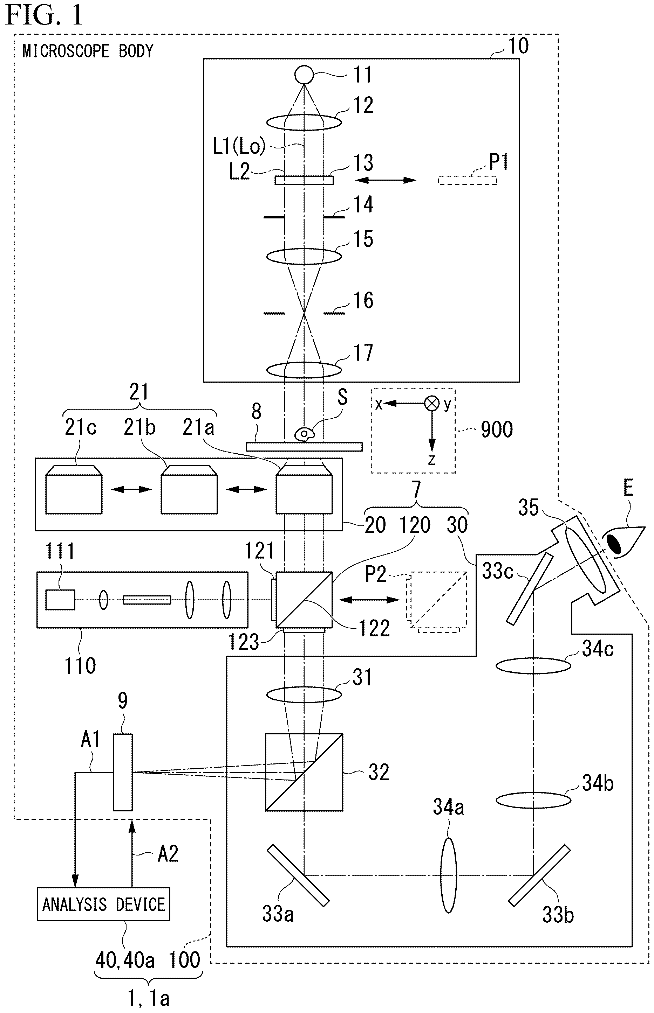

FIG. 1 is a conceptual diagram illustrating a configuration of an analysis system according to the embodiment. The analysis system 1 includes a microscope body 100 and an analysis device 40. The microscope body 100 is a microscope, that is, a quantitative phase microscope, and includes a transmissive illumination optical system 10 that emits illumination light to a sample S, a stage 8, an imaging optical system 7, a vertical illumination optical system 110, and a detection unit 9. The imaging optical system 7 includes an objective optical system 20, a filter cube 120, and a relay optical system 30.

The functions of the analysis device 40 may be provided in an electronic computer or the like which is physically separated from the microscope body 100.

The transmissive illumination optical system 10 includes a light source 11, a lens 12, a band-pass filter 13, a field diaphragm 14, a lens 15, an aperture diaphragm 16, and a condensing lens 17. The objective optical system 20 includes an objective lens 21. A plurality of objective lenses such as an objective lens 21a, an objective lens 21b, and an objective lens 21c can be selectively used as the objective lens 21. The relay optical system 30 includes an imaging lens 31, a beam splitter 32, a mirror 33a, a mirror 33b, a mirror 33c, a lens 34a, a lens 34b, a lens 34c, and an eyepiece 35. The eyepiece 35 is configured to allow looking thereinto with an eye E brought close thereto.

The number of objective lenses 21 interchangeable is not particularly limited. The form of an optical system included in the microscope body 100 is not particularly limited as long an image of a desired plane of a sample S can be acquired by imaging.

In FIG. 1, an optical axis of an optical system of the microscope body 100 is indicated by an alternate long and short dash line L1, and illumination light (luminous flux) from a light source 11 is indicated by an alternate long and two short dashes line L2. When a lens of which a focal point is disposed on a sample S out of the objective lenses 21 is an objective lens 21a, an optical axis Lo of the objective lens 21a which will be described later in detail matches the optical axis L1 of the microscope body 100, which is indicated by L1 (Lo) in FIG. 1. A flow of a detection signal of light detected by the detection unit 9 is indicated by a solid arrow A1, and control of the microscope body 100 by the analysis device 40 is schematically indicated by a solid arrow A2. The alternate long and short dash line L1, the alternate long and two short dashes line L2, the solid arrow A1, and the solid arrow A2 are the same in the following embodiment.

The light source 11 includes a non-coherent light source unit such as a halogen lamp and emits illumination light L2 which is applied to a sample S. In this embodiment, the light source 11 emits non-coherent light, and the emitted non-coherent light becomes light having a wavefront substantially perpendicular to an optical axis and enabling phase restoration by constituents of the transmissive illumination optical system 10 including the aperture diaphragm 16 which will be described later, which is applied to a sample S.

"Phase restoration" is to calculate a phase value of a sample S from an intensity of light from the sample S using an intensity transport equation. The illumination light L2 may be light of any wavelength as long as phase measurement of a sample S is possible, and it is preferable to use visible light without any change in view of visualization of a sample which is indistinct with visible light through phase measurement.

The illumination light L2 applied to a sample S may not be visible light, but may be ultraviolet light or infrared light. The wavefront of the illumination light L2 may not be substantially perpendicular to an optical axis as long as the shape of the wavefront is known, and the wavefront of the illumination light L2 may be, for example, substantially spherical. The light source 11 may include a coherent light source such as a pulse laser or a continuous wave (CW) oscillation laser and emit coherent light as the illumination light L2.

The illumination light L2 emitted from the light source 11 is incident on the lens 12. The illumination light L2 incident on the lens 12 is refracted by the lens 12, exits the lens 12 as a substantially parallel light, and is incident on the band-pass filter 13. Only light of a wavelength component in a predetermined wavelength range of the illumination light L2 incident on the band-pass filter 13 is transmitted by the band-pass filter 13 and is incident on the field diaphragm 14.

The wavelength range of light transmitted by the band-pass filter 13 is appropriately set such that an error between a measured value of a phase and an actual phase at the time of phase restoration (a phase restoration error which will be described later) which is caused by an axial chromatic aberration is not excessively large. The band-pass filter 13 is appropriately configured to be retractable to a position P1 outside of an optical path.

When a light source of which a wavelength range is narrow such as a laser beam is used as the light source 11, the band-pass filter 13 does not have to be disposed in the optical path. The wavelength range which is detected by a filter of the filter cube 120 disposed on an imaging side with respect to a sample S may be limited instead of using the band-pass filter 13.

The illumination light L2 incident on the field diaphragm 14 exits the field diaphragm 14 with a luminous flux diameter adjusted and is incident on the lens 15. The illumination light L2 incident on the lens 15 is converged by the lens 15, exits from the lens 15, and is incident on the aperture diaphragm 16. The illumination light L2 incident on the aperture diaphragm 16 is converted such that the wavefront thereof becomes a spherical shape, exits the aperture diaphragm 16, and is incident on the condensing lens 17. The illumination light L2 incident on the condensing lens 17 is refracted by the condensing lens 17, becomes light of which the wavefront is substantially perpendicular to the optical axis at the time of application to a sample S, and is applied to the sample S.

A sample S is disposed on the stage 8 such that a focal position of the objective lens 21a is included in a part on which phase measurement of the sample S is performed or in the vicinity thereof. The sample S is not particularly limited, but it is preferable that a rate of change in amplitude be relatively small and a rate of change in phase be relatively large when illumination light is applied to and transmitted by the sample S because an effect of phase measurement according to this embodiment is more considerably obtained. This object is referred to as a phase object. Particularly, a phase object such as cells like cultured cells is preferably used as the sample S in view of the above description.

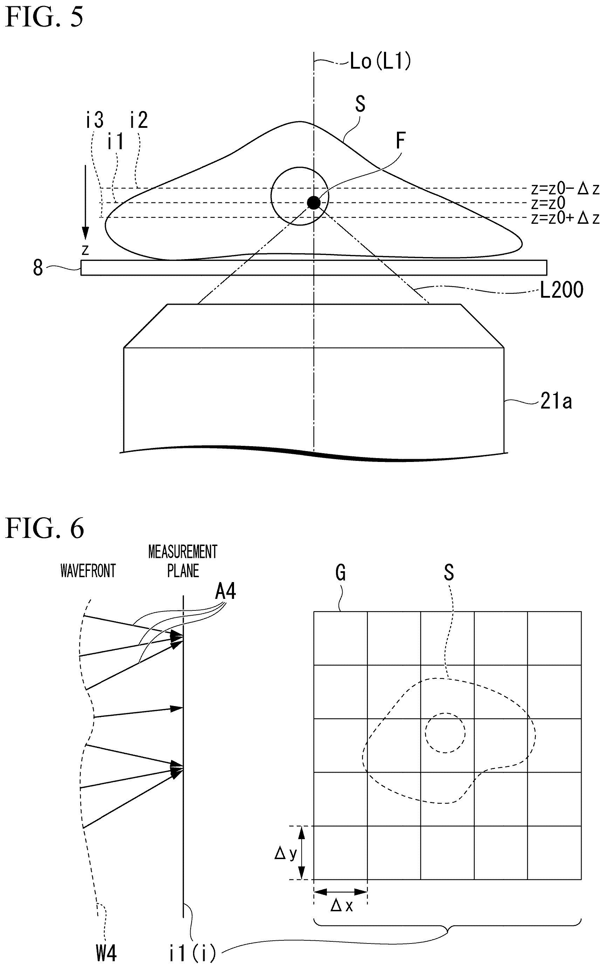

The stage 8 is configured to be movable along an optical axis of the objective lens 21a and an axis perpendicular to the optical axis. Here, the optical axis of the objective lens 21a is an axis Lo (see FIG. 5) represented by a straight line passing through an optical center of a lens surface on the sample S side of the objective lens 21a and a focal point of the objective lens 21a. The stage 8 is configured to be movable along the optical axis Lo of the objective lens 21a through electric driving using a moving mechanism such as a motor at the time of imaging the sample S.

In FIG. 5, a point at which the optical axis Lo of the objective lens 21a matches an optical axis L1 of the microscope body 100 is indicated by Lo(L1).

In the following embodiment, as indicated by coordinate axes 900 in FIG. 1, a z axis is set to be parallel to the optical axis L1 of the microscope body 100, that is, the optical axis Lo of the objective lens 21a, an x axis is set to be perpendicular to the z axis and parallel to the drawing surface, and a y axis is set to be perpendicular to the x axis and the z axis.

The objective optical system 20 includes the objective lens 21. In this embodiment, the objective lens 21a, the objective lens 21b, and the objective lens 21c are provided as the objective lens 21. As will be described later, the analysis system 1 is configured such that unevenness in obtained phase values is curbed when a sample S is imaged while interchanging the objective lens 21a, the objective lens 21b, and the objective lens 21c having different numerical apertures NA or the like and phase measurement is performed on the basis of data obtained from the objective lenses 21.

When a change in phase value measured when the objective lens 21 is interchanged is large, comparison between data acquired using different objective lenses 21 is difficult. In addition, since it is difficult to determine which objective lens 21 out of the different objective lenses 21 has been used to obtain data with the highest accuracy for restoring the phase, or the like, work related to a series of operations associated with measurement thereof becomes burdensome.

The vertical illumination optical system 110 includes a mercury lamp 111 and emits excitation light for fluorometry or the like. Light emitted from the vertical illumination optical system 110 is incident on the filter cube 120.

The filter cube 120 includes an excitation filter 121, a dichroic filter 122, and an emission filter 123. The filter cube 120 reflects light incident thereon from the vertical illumination optical system 110 to the sample S and transmits fluorescence from the sample S to the relay optical system 30. The filter cube 120 is configured to appropriately retract to a position P2 outside of the optical path.

In the excitation filter 121, a transmissive wavelength range is set such that the wavelength range of excitation light incident from the vertical illumination optical system 110 is set as a reflective wavelength range of the dichroic filter 122. Accordingly, the excitation filter 121 transmits excitation light of a partial wavelength range of the excitation light.

The dichroic filter 122 reflects the excitation light transmitted by the excitation filter 121 to the sample S and transmits fluorescence from the sample S incident from the objective lens 21a to the emission filter 123.

The absorption filter 123 absorbs unnecessary scattered light from the sample S or the optical system out of light incident from the dichroic filter 122 and emits only necessary light to the imaging lens 31 of the relay optical system 30.

The imaging lens 31 of the relay optical system 30 refracts light incident from the filter cube 120 to form an image on the detection unit 9 and emits the refracted light to the beam splitter 32.

The beam splitter 32 reflects some light incident from the filter cube 120 to the detection unit 9 and transmits the other light to the mirror 33a.

Light reflected to the mirror 33a is incident on the eyepiece 35 through reflection by mirrors or refraction by lenses in the order of the lens 34a, the mirror 33b, the lens 34b, the lens 34c, and the mirror 33c. Light incident on the eyepiece 35 is refracted by the eyepiece 35 and is incident on an eye E of a user and recognized by the user.

The detection unit 9 includes a detector of an imaging element such as a charge coupled device (CCD) or a complementary metal oxide semiconductor (CMOS) and detects light reflected by the beam splitter 32 of the relay optical system 30. A detection signal corresponding to the detected light is appropriately subjected to A/D conversion by an analog-to-digital (A/D) converter which is not illustrated or the like and is output to the analysis device 40. In other words, the detection unit 9 captures an image of the sample S.

In the analysis system 1, the detection unit 9 is configured to capture an image of the sample S irradiated with the illumination light L2 from the transmissive illumination optical system 10 at the time of phase measurement of the sample S, but the present invention is not limited thereto. For example, a configuration in which an image of a sample S irradiated with illumination light from the vertical illumination optical system 110 is captured may be employed. In this case, illumination light from the vertical illumination optical system 110 is applied to a sample S through the objective lens 21a (the objective optical system 20), and an image of the sample S is captured by the detection unit 9 on the basis of light reflected by the sample S.

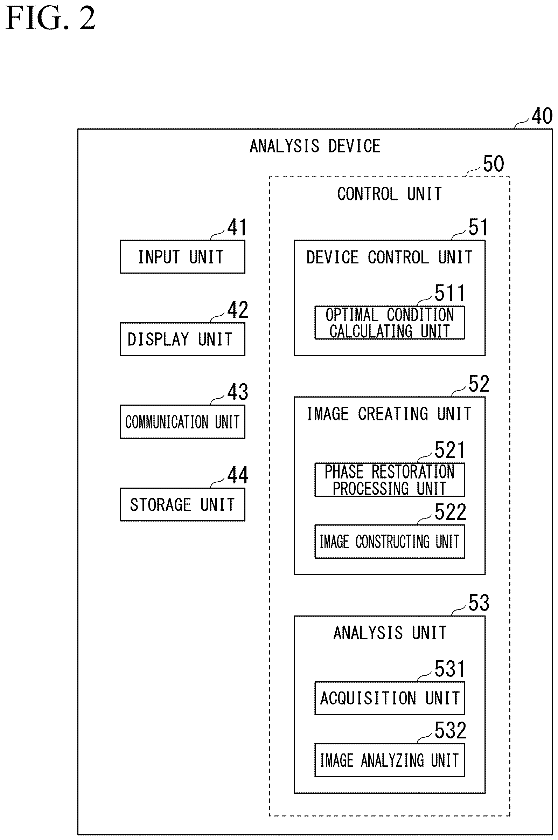

FIG. 2 is a diagram illustrating a configuration of the analysis device included in the analysis system according to this embodiment. The analysis device 40 includes an input unit 41, a display unit 42, a communication unit 43, a storage unit 44, and a control unit 50. The control unit 50 includes a device control unit 51, an image creating unit 52, and an analysis unit 53. The device control unit 51 includes an optimal condition calculating unit 511. The image creating unit 52 includes a phase restoration processing unit 521 and an image constructing unit 522. The analysis unit 53 includes an acquisition unit 531 and an image analyzing unit 532.

The input unit 41 is constituted by an input device such as a keyboard, a mouse, and/or a touch panel and receives input data including information required for analysis of data acquired through imaging of a sample S by the microscope body 100 or analysis of the data acquired through the imaging by the image creating unit 52.

An example of the information required for imaging of the sample S is information on a measurement scene. The input unit 41 appropriately stores the received input data in the storage unit 44 which will be described later. The input data may be acquired via the communication unit 43 which will be described later.

The display unit 42 is constituted by a display device such as a liquid crystal monitor and displays imaging conditions using the microscope body 100, a quantitative phase image created by the image creating unit 52 on the basis of data acquired through the imaging, analysis results generated by the analysis unit 53, and the like.

The communication unit 43 is constituted by a communication device for communication using a communication network such as the Internet and transmits imaging conditions using the microscope body 100, a quantitative phase image created by the image creating unit 52 on the basis of data acquired through the imaging, analysis results generated by the analysis unit 53, and the like or appropriately transmits and receives necessary data.

The storage unit 44 is constituted by a storage device such as a nonvolatile memory and stores a program causing the control unit 50 to perform processes, data required for imaging using the microscope body 100, data acquired through the imaging, a quantitative phase image created by the image creating unit 52 on the basis of the data, analysis results generated by the analysis unit 53, and the like.

The control unit 50 is constituted by a processor including a microprocessor such as a central processing unit (CPU) and serves as a main constituent of operations for controlling the analysis system 1. That is, by executing a program stored in the storage unit 44 or the like, the control unit 50 performs various processes such as a device control process of imaging a sample S using the microscope body 100, an image creating process such as a phase restoration process for data acquired through the imaging, an image analyzing process, and an output process.

The device control unit 51 of the control unit 50 appropriately calculates parameters required for imaging a sample S using the microscope body 100 on the basis of input data input by a user and controls operations of the constituents of the microscope body 100 on the basis of the acquired parameters, the input data, and the like.

The optimal condition calculating unit 511 calculates an interval $\Delta z$ among the plurality of positions which are set when the microscope body 100 arranges a focal point of the objective lens 21a at a plurality of positions on a sample S and detects light from the sample S using the detection unit 9. This interval $\Delta z$ is referred to as a focal interval. The focal interval $\Delta z$ is a parameter important to accurately performing phase measurement. This will be described below on the basis of a phase measurement method using an intensity transport equation.

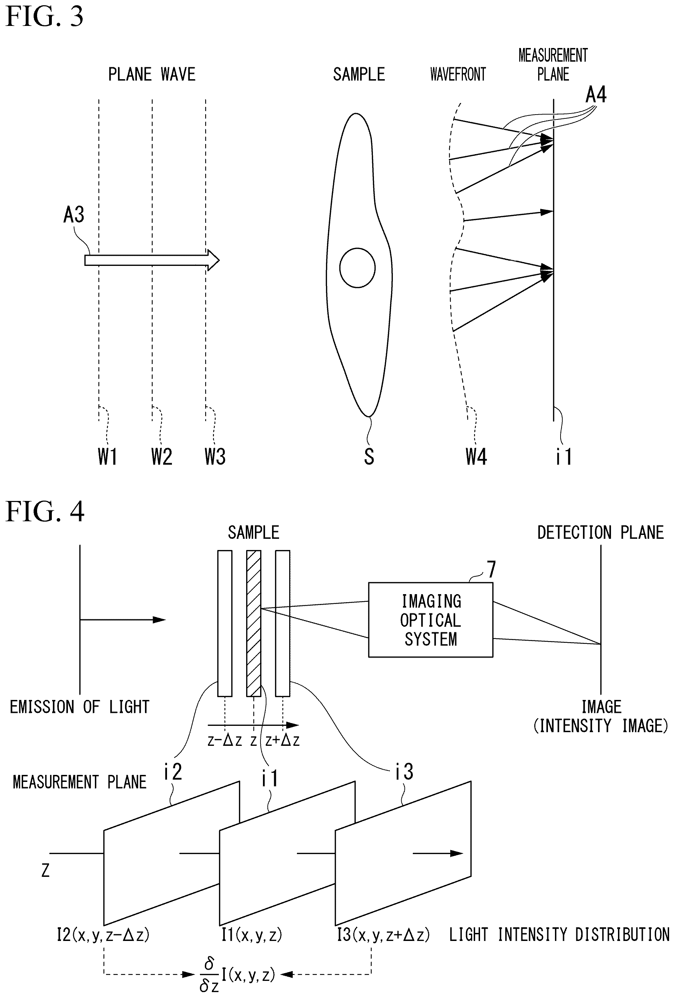

FIG. 3 is a conceptual diagram illustrating change of an intensity distribution of light transmitted by a phase object. A sample S is a phase object such as a cell, and the sample S is irradiated with plane waves having wavefronts parallel with each other and substantially perpendicular to an optical axis as schematically indicated by wavefronts W1, W2, and W3. A progressing direction of the plane waves is indicated by an arrow A3.

Light transmitted by the sample S does not change greatly in amplitude, but an equi-phase plane, that is, a wavefront, changes with change of a phase thereof (a wavefront W4). In the Huygens-Fresnel principle, light constituting a curved wavefront reaches a measurement plane i1 as indicated by a solid arrow A4.

Here, as will be described later in detail, for example, a specific plane of the sample S on which the objective lens 21a is focused (that is, an in-focus plane of the objective lens 21a) is referred to as a measurement plane i1.

The measurement plane i1 is a plane substantially perpendicular to the optical axis of the objective lens 21a. In FIG. 3, the measurement plane i1 is substantially parallel to the wavefronts W1 to S3 of the plane waves. On the measurement plane i1, an intensity distribution of predetermined light (hereinafter referred to as a light intensity distribution) is formed with change of the wavefronts due to the sample S as described above.

A method of measuring change in phase by analyzing the intensity of transmitted light on the basis of a relationship between change of a phase of the transmitted light transmitted by the sample S and the light intensity distribution has been proposed. In this embodiment, for example, a method using an intensity transport equation is used. A relationship between an intensity I and a phase ($\phi$) in propagating waves is described by an intensity transport equation.

FIG. 4 is a diagram illustrating a method of detecting light from a sample S which is performed to calculate a phase distribution $\phi$. First, it is necessary to acquire a light intensity distribution on the measurement plane i 1 of the sample S and a differential coefficient distribution with respect to z on the measurement plane i1. Accordingly, as illustrated in FIG. 4, the device control unit 51 sets the focal point of the objective lens 21a to be included in a desired measurement plane i1, causes the imaging optical system 7 to form an image of the sample S on the detection plane of the detection unit 9, and causes the detection unit 9 to detect light from the sample S.

Detection signals from pixels of the detection unit 9 are input to the control unit 50 (FIG. 2), and light intensity distribution data in which positions of the pixels and light intensities based on the detection signals are correlated is generated by the phase restoration processing unit 521.

The light intensity distribution data is data representing an intensity distribution of light detected by a pixel of the detection unit 9 corresponding to a position of coordinates (x, y) on the measurement plane corresponding to a value of a certain coordinate z. The light intensity distribution data is data representing a light intensity corresponding to a value of the coordinates x, y, and z and is constructed in the form of a lookup table.

For example, by distinguishing the light intensity distribution on the measurement plane corresponding to values of certain coordinates z in colors or gradations and two-dimensionally mapping the light intensity distribution, it is possible to create an image indicating an intensity distribution of light at the z positions (hereinafter referred to as a light-intensity image).

A data structure of the light intensity distribution data is not particularly limited as long as a value of a light intensity corresponding to a value of predetermined coordinates x, y, and z can be taken, and an existing other data structure may be employed.

It is preferable that a position of the measurement plane i 1 (that is, a focal position of the objective lens 21a) be set to a position based on contrast of a light intensity of light from the sample S detected by the detection unit 9. The device control unit 51 can set the position of the measurement plane i1 on the basis of parameters calculated from three-dimensional light intensity distribution data of the sample S (parameters indicating contrast of light intensities of light from the sample S such as a variance vz which will be described later) acquired in advance (before phase measurement according to this embodiment is performed).

When the three-dimensional light intensity distribution data is acquired in advance through imaging, the focal point on the sample S at the time of imaging for acquiring the light intensity distribution data is not particularly limited and can be appropriately set.

When a two-dimensional light intensity distribution Iz(x, y) in the x direction and the y direction corresponding to each value of z in the three-dimensional light intensity distribution data acquired in advance is defined as Iz(x, y), the device control unit 51 calculates variance value vz of the light intensity distribution corresponding to each value of z.

The device control unit 51 sets the position of the measurement plane i1, that is, the focal position of the objective lens 21a, on the basis of the calculated variance values vz corresponding to the values of z. For example, the device control unit 51 sets the position of the measurement plane i1 to the position in the z direction corresponding to a minimum variance vz out of the calculated variances vz. In other words, the device control unit 51 sets the position of the measurement plane i1 to the value of z corresponding to the light intensity distribution data with low contrast.

The device control unit 51 sequentially sets the focal position on the measurement plane i2 and the measurement plane i3 such that the focal position of the objective lens 21a becomes positions deviated by a distance $-\Delta z$ and $+\Delta z$ from the position on the measurement plane i1 along the z axis on the basis of the focal interval $\Delta z$ calculated by the optimal condition calculating unit 511. When the focal point of the objective lens 21a is disposed on the measurement plane i2 and the measurement plane i3, the device control unit 51 causes the imaging optical system 7 to form an image of the sample S on the detection plane of the detection unit 9 and causes the detection unit 9 to detect light from the sample S.

Detection signals from the pixels of the detection unit 9 corresponding to the focal points of the objective lens 21a disposed on the measurement plane i2 and the measurement plane i3 are input to the control unit 50 (FIG. 2), and light intensity distribution data in which the positions of the pixels are correlated with the light intensities based on the detection signals is generated by the phase restoration processing unit 521.

As long as the positions of the focal points of the objective lens 21a disposed on the measurement planes i1 to i3 are present on the measurement planes i1 to i3, the position on the XY plane is not particularly limited.

The generated light intensity distribution data corresponding to the measurement plane i2 and the measurement plane i3 is used to calculate a differential coefficient of the light intensity on the measurement plane i1 with respect to z.

The phase restoration processing unit 521 calculates dI/dz=(I3-I2)/2$\Delta$z corresponding to the differential coefficient of the light intensity on the measurement plane i1 with respect to z by dividing a difference between intensity values of two points including a point on the measurement plane i2 and a point on the measurement plane i3 and located at corresponding positions such as positions with the same coordinates on the XY plane by 2$\Delta$z which is a distance between the measurement plane i2 and the measurement plane i3.

The phase restoration processing unit 521 appropriately stores data corresponding to the calculated differential coefficient distribution of the light intensity with respect to z (hereinafter referred to as differential coefficient distribution data) in the storage unit 44 or the like. The differential coefficient distribution data is data indicating a distribution of differential coefficients of the light intensity corresponding to the values of coordinates x, y, and z with respect to z and is constructed in the form of a lookup table.

As long as values of the differential coefficients corresponding to values of predetermined coordinates x, y, and z with respect to z can be taken, the data structure of the differential coefficient distribution data is not particularly limited and another existing data structure may be employed.

FIG. 5 illustrates an arrangement example of the focal point of the objective lens 21a and the measurement planes i1 to i3 at the time of imaging a sample S. In FIG. 5, a sample S is placed on the stage 8. In FIG. 5, the z axis is set to be parallel to the optical axis Lo of the objective lens 21a, and a direction directed from the sample S to the objective lens 21a is defined as a plus (+) direction of the z axis. Aluminous flux of light incident on the objective lens 21a from the focal position F is schematically indicated by an alternate long and two short dashes line L200. In FIG. 5, the focal position F of the objective lens 21a is set to z=z0, and a plane including the point of z=z0 and perpendicular to the z axis is defined as the measurement plane i1.

The device control unit 51 moves the stage 8 through electric driving using a moving mechanism such as a motor and sets the focal position of the objective lens 21a on the measurement plane i1, the measurement plane i2, and the measurement plane i3. The device control unit 51 sequentially arranges the focal point of the objective lens 21a at a plurality of positions separated by the focal interval $\Delta$z from each other along the optical axis Lo of the objective lens 21a.

Here, a "plurality of positions separated by the focal interval $\Delta$z from each other along the optical axis Lo of the objective lens 21a" indicates a plurality of positions separated by a distance of $\Delta$z from each other when the plurality of positions are projected onto the optical axis Lo of the objective lens 21a. Since, the objective lens 21a is located on the stage side 8 of the imaging optical system 7, the focal position F of the objective lens 21a can be replaced with a focal position of the objective optical system 20 or the imaging optical system 7.

The optimal condition calculating unit 511 of the device control unit 51 calculates the focal interval $\Delta$z using setting information of the microscope body 100 in this embodiment and a parameter k which will be described later in detail. Here, the setting information of the microscope body 100 is information which is set in the microscope body 100 to generate a quantitative phase image and is, for example, information such as a numerical aperture of the objective lens 21a, a wavelength of illumination light L2, and a refractive index between the objective lens 21a and the sample S.

When the objective lens 21a is a dry objective lens, the atmosphere between the objective lens 21a and the sample S is air, and thus the device control unit 51 sets, for example, n=1.00 as a refractive index of air. On the other hand, when the objective lens 21a is an immersion objective lens, the device control unit 51 sets a refractive index of an immersion liquid filled between the objective lens 21a and the sample S as a refractive index n.

NA is a numerical aperture of the objective lens 21a and is set on the basis of a user's input to the input unit 41 or the like by the device control unit 51. The optimal condition calculating unit 511 calculates the focal interval $\Delta$z on the basis of the setting information of the microscope body 100. Specifically, the optimal condition calculating unit 511 calculates the focal interval $\Delta$z on the basis of information such as the numerical aperture NA of the objective lens 21a, the wavelength $\lambda$ of the illumination light L2, and the refractive index n between the objective lens 21a and the sample S.

The parameter k has a value equal to or greater than 1, and phase restoration can be performed up to a cutoff spatial frequency at which performance of the objective lens 21a is maximized using the intensity transport equation. As the value of k becomes greater than 1, the spatial frequency at which phase restoration using the intensity transport equation can be performed becomes lower. In the following description, the parameter k is referred to as a phase restoration parameter.

A difference between an actual phase value and a measured phase value of the sample S is referred to as a phase restoration error. When phase restoration is performed at a high spatial frequency, the phase restoration error basically decreases. When the phase restoration error is small, a phase closer to the actual phase value (that is, a phase with higher accuracy) is restored and thus it can be said that accuracy of phase restoration (hereinafter referred to as phase restoration accuracy) is high.

The optimal condition calculating unit 511 preferably sets the phase restoration parameter k to equal to or less than 25 and more preferably sets the phase restoration parameter k to equal to or less than 16. When the phase restoration parameter k increases, the spatial frequency at which phase restoration is possible is lowered as described above. When the focal interval $\Delta z$ at k=25 is employed, the performance corresponds to phase restoration up to a spatial frequency which is 1/5 of the cutoff spatial frequency of the objective lens 21a.

When the focal interval $\Delta z$ at k=16 is employed, the performance corresponds to phase restoration up to a spatial frequency which is 1/4 of the cutoff spatial frequency of the objective lens 21a. In observing a sample S (a phase object) such as cells with a size of several $\mu$m to several hundreds of $\mu$m in visible light, it is preferable that the phase restoration parameter k be set to a relatively large value equal to or less than 25 in order to detect the whole cells, and it is more preferable that the parameter K be set to equal to or less than 16 in order to easily detect a relatively large structure such as a nucleus in a cell.

In the example, the value of the parameter k is determined to correspond to phase restoration up to a spatial frequency which is 1/5 or 1/4 of the cutoff spatial frequency of the objective lens 21a, but the value of the parameter k is not limited thereto and can be set according to a resolution required for phase measurement of the sample S.

The optimal condition calculating unit 511 may set the focal interval $\Delta z$ on the basis of a point intensity distribution (point spread function (PSF)) based on the objective lens 21a. The optimal condition calculating unit 511 can set the focal interval $\Delta z$ in a range in which k is equal to or less than 5, for example, on the basis of the condition of k=5 which is the value of k when a position of a first dark ring of the point intensity distribution (PSF) is used as a reference.

For example, the optimal condition calculating unit 511 may set the focal interval $\Delta z$ in a range in which k is equal to or greater than 1 and equal to or less than 5 by adding a condition (k=1) in which phase restoration is possible up to the cutoff spatial frequency of the objective lens 21a as described above.

The optimal condition calculating unit 511 can perform phase restoration up to a high spatial frequency with high accuracy by setting the focal interval $\Delta z$ on the basis of a distance between the focal position F and the position of the first dark ring. When the objective lens 21 is interchanged, the phase value at the time of phase restoration can be kept substantially constant by changing the focal interval $\Delta z$ according to the numerical aperture NA or the like of the objective lens 21 while fixing the phase restoration parameter k.

For example, a cell structure or the like with a size corresponding to a frequency region in which phase restoration can be performed when the numerical aperture NA of the objective lens 21 is 0.3 is calculated as the same phase value even when the objective lens 21 is interchanged with an objective lens of which the numerical aperture NA is 0.75 or 0.95. Accordingly, even when the objective lens 21 is interchanged for the same sample S, unevenness in phase acquired as the result of phase restoration decreases and it is possible to quantitatively analyze the sample S.

As described above, accuracy of phase restoration can be increased by adjusting the value of the focal interval $\Delta z$, but the accuracy of phase restoration can be increased by adjusting a sampling interval on the sample S in addition to the focal interval $\Delta z$.

FIG. 6 is a diagram illustrating a sampling interval. As described above with reference to FIG. 3, the wavefront W4 which has become non-planar by causing the wavefront to be transmitted by the sample S forms light intensity distributions based on change in phase on the measurement planes i1 to i3 (in the following description, the measurement planes i1 to i3 are collectively referred to as measurement planes i). The right part of FIG. 6 is a conceptual diagram of the measurement plane i when seen from the optical center of the objective lens 21a and illustrates parts G corresponding to the individual pixels of the detection unit 9.

The parts G corresponding to the individual pixels are arranged to cover a region corresponding to a sample S in a grid shape. In the following embodiment, the sampling intervals $\Delta x$ and $\Delta y$ are a width of the part G corresponding to the individual pixel on the measurement plane i on which the focal position of the objective lens 21a is set in this way.

The sampling intervals $\Delta x$ and $\Delta y$ satisfy $\Delta x = \Delta y = P/\beta$ when a pixel size of the detection unit 9 is defined as P and a lateral magnification of the imaging optical system 7 is defined as $\beta$. Here, the pixel size of the detection unit 9 indicates a width of each pixel in case of CCD or the like, and the pixel size P is a size obtained by dividing a field number (diagonal) by a scan magnification in case of a scanning microscope using a laser-scanning fluorescence observation unit which will be described later in Modified Example 6. In the following description, a sampling interval is described as $\Delta x$. The lateral magnification $\beta$ of the imaging optical system 7 is a product of a magnification $\beta 1$ of the objective lens 21a and a magnification $\beta 2$ of the relay optical system 30.

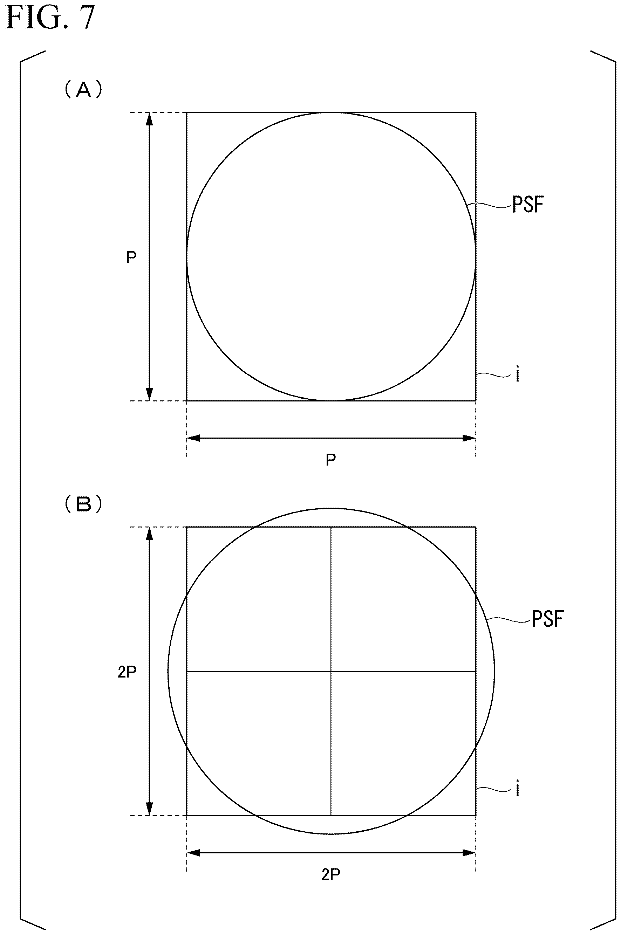

FIG. 7(A) is a conceptual diagram illustrating a method of setting a sampling interval $\Delta x$. The optimal condition calculating unit 511 sets the magnification $\beta$ of the imaging optical system 7 on the basis of a size of an Airy disk of the point intensity distribution (PSF). In FIG. 7(A), an example in which a diameter of the Airy disk of the point intensity distribution (PSF) projected onto the detection plane of the detection unit 9 is the same as the width of the pixel size P is schematically illustrated. It is preferable that the optimal condition calculating unit 511 set the magnification $\beta$ of the imaging optical system 7 such that the diameter of the Airy disk of the point intensity distribution (PSF) projected onto the detection plane of the detection unit 9 is larger than the width of the pixel size P.

Alternatively, it is preferable that the optimal condition calculating unit 511 set the magnification $\beta$ of the imaging optical system 7 such that the diameter of the Airy disk of the point intensity distribution (PSF) projected onto the detection plane of the detection unit 9 is larger than the area of the pixel size P. In this case, the sampling interval $\Delta x = P/\beta$ is set on the basis of the wavelength $\lambda$ of illumination light and the numerical aperture NA of the objective lens 21a.

FIG. 7(B) is a conceptual diagram illustrating a method of setting the sampling interval $\Delta x$. It is particularly preferable that the optimal condition calculating unit 511 set the magnification $\beta$ of the imaging optical system 7 such that the area of the Airy disk of the point intensity distribution (PSF) projected onto the detection plane of the detection unit 9 is four times to five times the area of the pixel size P. Accordingly, it is possible to further curb a decrease in accuracy of phase restoration due to noise of the light intensity distributions I1, I2, and I3.

When the optimal condition calculating unit 511 sets the number of positions at which the focal point of the objective lens 21a is disposed and the focal interval $\Delta z$ as described above, the device control unit 51 images the sample S on the basis of the set number of positions at which the focal point of the objective lens 21a is disposed and the set focal interval $\Delta z$. A detection signal obtained by causing the detection unit 9 to detect light from the sample S is input to the phase restoration processing unit 521 (FIG. 2) of the image creating unit 52.

The image creating unit 52 processes the detection signal input from the detection unit 9. The image creating unit 52 serves as an image creating unit that generates light intensity distribution data from the detection signal and creates a quantitative phase image on the basis of the light intensity distribution data. The analysis unit 53 performs analysis on the basis of the phase of the sample S which has been restored on the basis of the light intensity distribution data.

The phase restoration processing unit 521 of the image creating unit 52 generates light intensity distribution data in which the positions of the pixels of the detection unit 9 are correlated with light intensities based on the input detection signal and appropriately stores the generated light intensity distribution data in the storage unit 44 or the like. The phase restoration processing unit 521 generates data (hereinafter referred to as phase distribution data) corresponding to a phase distribution $\phi$ of the sample S including the measurement plane i1 or the like from the generated light intensity distribution data on the basis of the intensity transport equation and the focal interval $\Delta z$ set by the optimal condition calculating unit 511 and appropriately stores the generated data in the storage unit 44 or the like. An example of the phase distribution data is data in which phases are correlated with the values of coordinates x, y, and z and is constructed in the form of a lookup table.

As long as a phase value corresponding to the value of predetermined coordinates x, y, and z can be taken, the data structure of the phase distribution data is not particularly limited, and another existing data structure may be employed.

When the optimal condition calculating unit 511 sets the number of positions at which the focal point of the objective lens 21a is disposed to a value greater than 3, it is preferable that the phase restoration processing unit 521 restore the phase from the light intensity distribution data using a Savitzky-Golay method (hereinafter referred to as an SG method).

The image constructing unit 522 (FIG. 2) constructs a two-dimensional or three-dimensional quantitative phase image on the basis of the phase distribution data generated by the phase restoration processing unit 521 and stores the generated quantitative phase image in the storage unit 44 or the like. Here, the quantitative phase image is an image representing (visualizing) a phase which is a product of thickness in the sample and a change in refractive index.

Specifically, for example, the quantitative phase image is an image in which gradation values are set according to the phase values calculated using the intensity transport equation. For example, the quantitative phase image is an image in which grayscale values of gradations proportional to the magnitudes of the phase values. In this case, in the quantitative phase image, height information on a thickness (height) in the z direction of the sample S can be viewed from the gradation values (the magnitudes of the phase values).

In addition, the image constructing unit 522 constructs a phase difference image on the basis of the phase distribution data or the quantitative phase image and the light intensity distributions I1, I2, and I3. In the following description, the phase difference image acquired through the calculation process in the image constructing unit 522 is referred to as a quantitative phase image. The quantitative phase image is not limited to the image in which the gradation values are set according to the phase values calculated using the intensity transport equation and may be an image in which the gradation values are set according to the phase values calculated using another known method.

The acquisition unit 531 acquires the quantitative phase image created by the image creating unit 52. An example of the quantitative phase image includes an image of a plurality of cultured cells. In order to increase the number of dead cells in the cultured cells, Chinese Hamster ovary (CHO)-S cells were cultured in a glucose-free medium. Since there is no glucose in the medium, the cells fall into a starved state and thus the number of dead cells can be increased for a relatively short time.

A container used to observe cells will be described below.

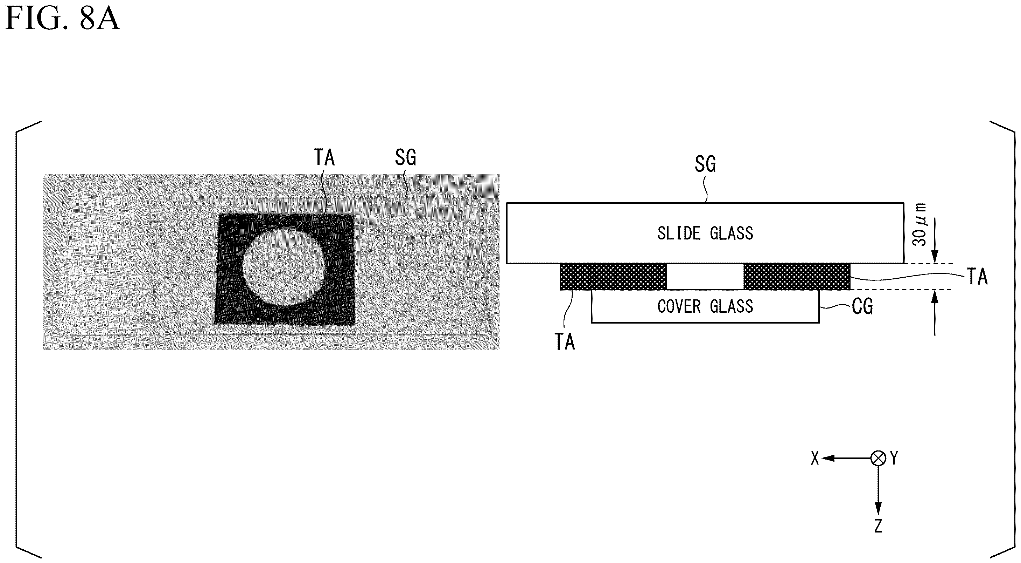

FIG. 8A is a diagram illustrating Example 1 of a container. Since cells to be evaluated are floating cells, sedimentation of cells occurs in a container such as a 96-well plate with the elapse of time. A problem in which cells are widely distributed in the depth direction (an optical-axis direction) of the medium and the focal point is difficult to determine occurs, which is not desirable for observation. Therefore, a tape in which an opening of $\phi$15 mm was formed in a black double-sided tape TA with a thickness of 30 μm was attached to a slide glass SG. A container in which a liquid layer of the cell suspension is 30 μm is formed by dropping a cell suspension (a mixture of cells and a medium) on the opening of φ15 mm and covering it with a cover glass CG from above.

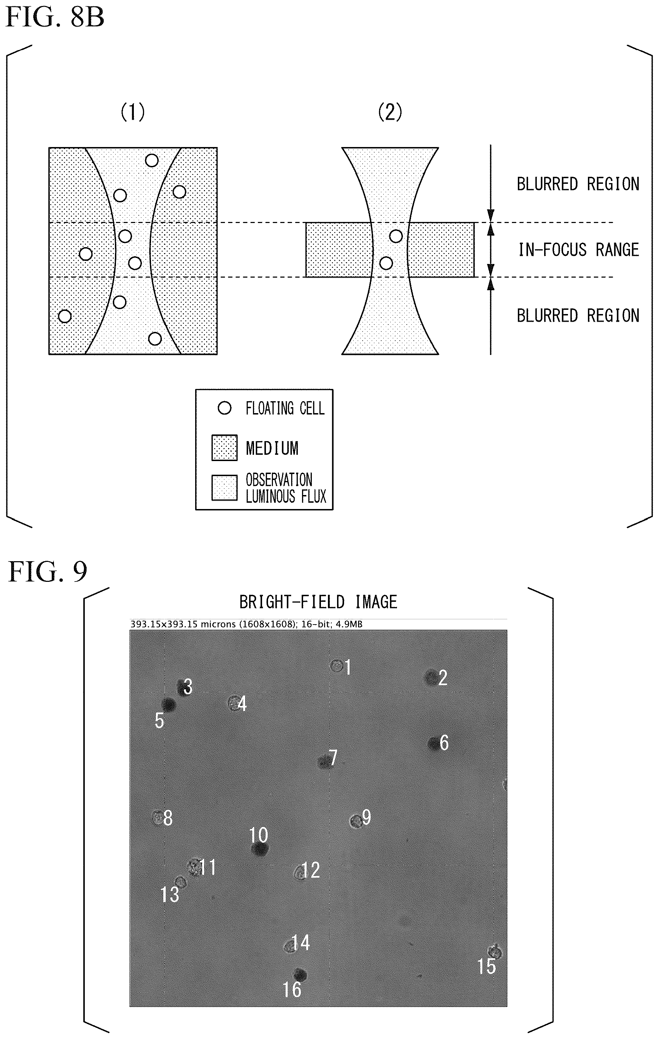

FIG. 8B is a diagram illustrating Example 2 of the container. In FIG. 8B, (1) indicates a container according to the related art (for example, a 96-well plate), and (2) indicates a thin-layer container (a thin-layer cell) which is thinner than the container of (1) in FIG. 8B.

In the thin-layered container illustrated in FIG. 8A, since the thickness between the slide glass SG and the cover glass CG is small, there are merits that an influence of sedimentation of cells is small (there is no change of the focal position even when left one day) and the number of cells staying in an in-focus range of the observation optical system (the objective lens) is increased. Since the number of cells hard to cause sedimentation of cells with long-term observation due to the thickness of the accommodation unit and staying at the focal position can be increased by observing floating cells in the thin-layered cell of (2) illustrated in FIG. 8B, excellent observation is possible. In (1) and (2) of FIG. 8B, the slide glass SG and the cover glass CG of the top and bottom of the medium of the cell suspension in the accommodation unit are not illustrated.

Floating Chinese hamster ovary (CHO) cells were used as the cells. By culturing the cells in a glucose-free medium as a cell medium for 18 hours, the cell survival rate could be decreased for a relatively short time.

The cell suspension cultured in the glucose-free medium was dispensed into micro test tubes by 10 μL. Then, 0.5% trypan blue stain widely used to determine life and death of cells was dispensed by 10 μL and dropped on the micro test tubes containing the cell suspension, and then pipetting was performed. 5.3 μL was dispensed from the micro test tubes in which the cell suspension and the trypan blue stain were mixed, dropped on the thin-layered observation container illustrated in FIG. 8A, covered with a cover glass, and placed on the stage 8.

Bright-field observation was performed using the microscope body 100 and using a 20x objective lens (NA 0.75), a zoom magnification 1.5x, and a white light emitting diode (LED) light source as a transmission illumination. Three images shifted 5 μm forward and backward from the focal position in addition to an image of the focal position were acquired to generate a quantitative phase image. A quantitative phase image was constructed by acquiring three bright-field images including a focal image and the images different therefrom in the position Δz.

FIG. 9 is a diagram illustrating an example of the bright-field image. FIG. 9 illustrates a bright-field image at the focal position, and numbers are added to distinguish individual cells. Cells in black in FIG. 9 are dead cells stained with trypan blue stain.

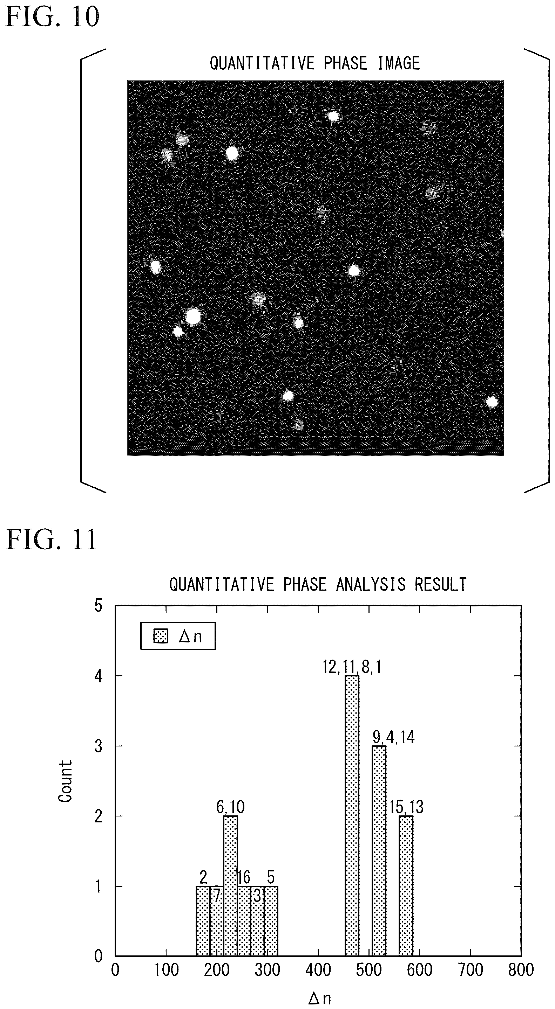

FIG. 10 is a diagram illustrating an example of a quantitative phase image. FIG. 10 illustrates a quantitative phase image constructed from the bright-field image illustrated in FIG. 9 and the images shifted 5 μm forward and backward from the focal position. As can be seen in FIG. 10, cell images having different luminance values are observed. In FIG. 10, dead cells corresponding to "2," "3," "5," "6," "7," "10," and "16" in FIG. 9 are observed. Description will be continued with reference back to FIG. 2.

The image analyzing unit 532 acquires the quantitative phase image from the acquisition unit 531. The image analyzing unit 532 derives a refractive index difference (Δn) between a medium and a cell in living cells included in the acquired quantitative phase image.

A method of calculating a refractive index difference between a cell and a medium from a quantitative phase image will be described below.

Cell diameter information is required for calculating a refractive index difference between a cell and a medium. An example in which a method of calculating a cell diameter from a cell area is employed will be described here.

$$\text{Cell diameter} = 2 \times \sqrt{(\text{cell area}/\pi)} \qquad \ldots (1)$$

When an observed cell image is separated from a circle, the cell diameter cannot be accurately calculated. Accordingly, a cell with high roundness is selected. Since the method of deriving a cell diameter can be performed using a technique other than Expression (1), it is not essential to determine a roundness threshold value. Since cells used herein are floating cells, most of the cells are spherical in a medium.

Then, the quantitative phase image was binarized to form cell body masks. Cells laid across an edge of an image may be observed in a certain observation image. In this case, since a cell diameter cannot be accurately calculated, cell body masks present on an image edge are excluded. Roundness of each resultant cell body mask is calculated, and the cell diameter is calculated from areas of the cell body masks of which the roundness is equal to or greater than a predetermined roundness threshold value using Expression (1).

Then, the generated cell body masks are overlapped on the quantitative phase image illustrated in FIG. 10, and a maximum luminance value of the quantitative phase image is calculated from the cell bodies detected in the range of the cell body masks. An integral value of the quantitative phase image in the cell body masks may be calculated instead of the maximum luminance value. Then, refractive index difference information between a cell and a medium is calculated by dividing the acquired maximum luminance value by the cell diameter. The luminance values of the quantitative phase image indicate phase values. A phase value is expressed by Expression (2).

$$\text{Phase value} = (2 \times \pi \times \Delta n \times \text{cell thickness})/\lambda \qquad \ldots (2)$$

Here, $\lambda$ denotes an observation wavelength, and $\Delta n$ denotes a refractive index difference between a cell and a medium. Accordingly, refractive index difference information between a cell and a medium can be acquired by dividing the maximum luminance value in the cell masks in the quantitative phase image by the cell diameter (a cell thickness).

FIG. 11 is a diagram illustrating an example of a quantitative phase analysis result in the analysis system according to this embodiment. In the histogram illustrated in FIG. 11, the vertical axis represents grades such as refractive index differences ($\Delta n$), and the vertical axis represents frequencies such as counts. In FIG. 11, numbers corresponding to the cell body numbers in FIG. 9 are added.

As can be seen from FIG. 11, the histogram is divided with respect to a refractive index difference ($\Delta n$) of around 400 and is divided into a group with large refractive index differences ($\Delta n$) and a group with small refractive index differences ($\Delta n$). The image analyzing unit 532 determines a threshold value for the refractive index differences (hereinafter referred to as a "living-dead determination threshold value") for determining whether a cell is living or dead on the basis of the quantitative phase analysis result.

Specifically, at least three bright-field images (a focal image, a pre-focal image, and a post-focal image) are captured, and a quantitative phase image is constructed from changes in pixel luminance in the images. A luminance value in the quantitative phase image has information proportional to cell thickness $\times$ (refractive index difference between cell and medium). Protein or amino acid which is a constituent element thereof has a higher molar refractive index than water. When an amount of protein or amino acid which is a constituent element thereof is large, it can be considered that the luminance value of the quantitative phase image is high and cells thereof are activated, and it is also predicted that an antibody production capacity is high.

Distinction of living cells and dead cells based on the living-dead determination threshold value of the refractive index differences ($\Delta n$) will be described below.

The luminance value of the quantitative phase image is proportional to the phase value and a magnitude thereof is represented by a product of the refractive index difference between a cell and a medium and a cell thickness. Focus is put on only the refractive index differences ($\Delta n$) between a cell and a medium by dividing the luminance value of the phase distribution by the cell thickness.

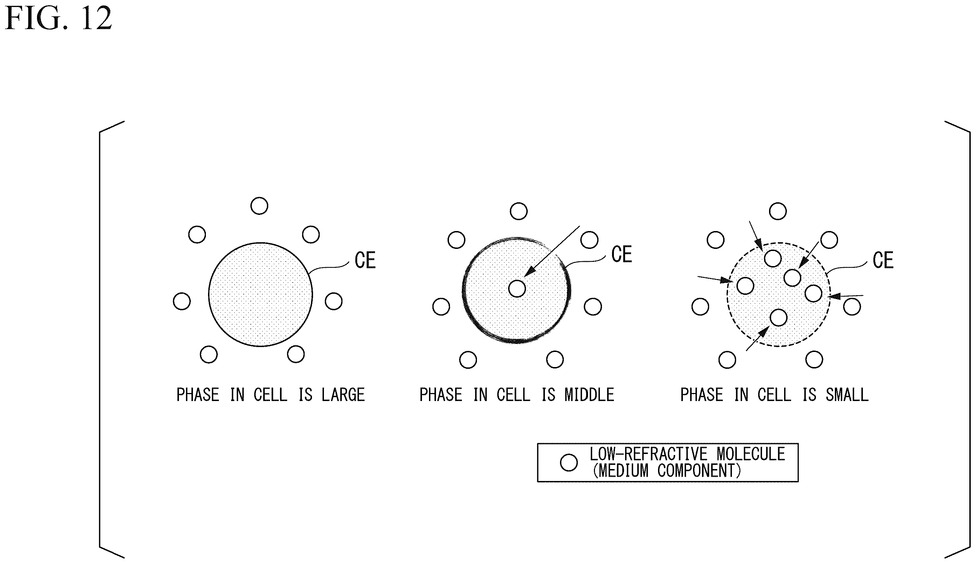

FIG. 12 is a diagram illustrating the refractive index difference between a cell and a medium. A case in which a phase of a cell is large, a case in which a phase of a cell is middle, and a case in which a phase of a cell is small will be described below.

As illustrated in the left part of FIG. 12, when a phase of a cell is large, a cell membrane maintaining homeostasis of the cell CE is not broken and thus the refractive index difference between the cell and a medium increases. As illustrated in the middle part of FIG. 12, when a phase of a cell is middle and the cell is weakened, the homeostasis of the cell CE cannot be maintained and thus a cell membrane is partially broken. When the cell membrane is broken, a medium component invades into the cell. Since a refractive index of the medium component is smaller than the refractive index of the cell (substantially equal to the refractive index of water), the refractive index of the cell decreases when the cell membrane is broken and the medium invades into the cell. Accordingly, the refractive index difference between the cell and the medium becomes intermediate. As illustrated in the right part of FIG. 12, when a phase of a cell is small, the cell membrane maintaining the homeostasis of the cell CE is broken and thus the refractive index difference between the cell and the medium decreases, When the cell membrane is fully broken and the medium invades into the cell, the refractive index difference ($\Delta n$) becomes zero. This serves as a mechanism for distinguishing a living cell and a dead cell.

The image analyzing unit 532 binarizes the quantitative phase image and generates cell body masks. The image analyzing unit 532 compares the cell body masks with the bright-field images and determines a binarization threshold value with which a cell body mask matches the outline of a cell body in the bright-field images.

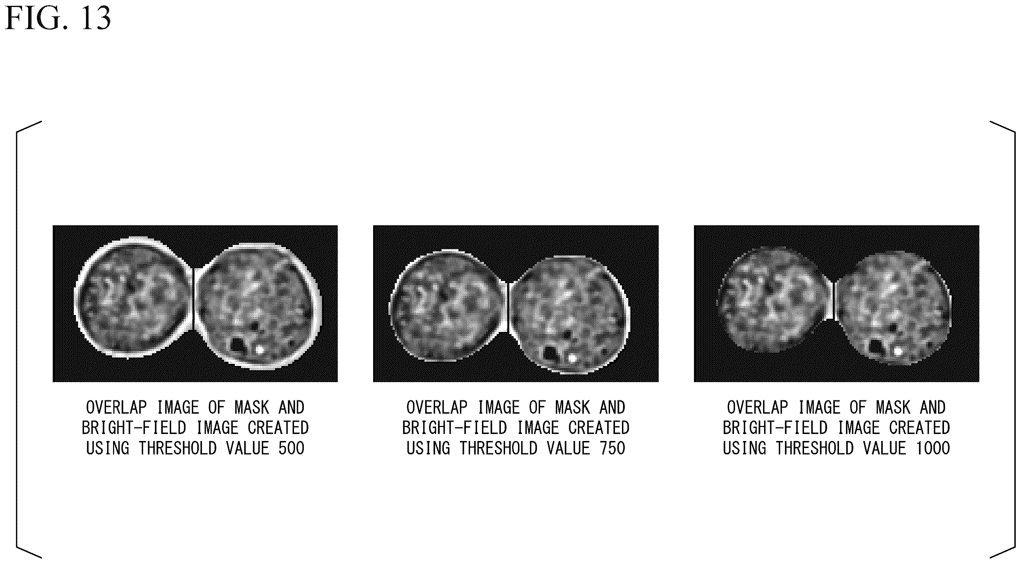

FIG. 13 is a diagram illustrating a method of determining the binarization threshold value. In FIG. 13, the left part illustrates an image in which a cell body mask generated using a binarization threshold value 500 overlaps a bright-field image, the middle part illustrates an image in which a cell body mask generated using a binarization threshold value 750 overlaps a bright-field image, and the right part illustrates an image in which a cell body mask generated using a binarization threshold value 1000 overlaps a bright-field image.

In FIG. 13, the cell body masks generated using the binarization threshold value 500 and the binarization threshold value 750 show a clearance on the circumference of a cell. The cell body mask generated using the binarization threshold value 1000 does not show a clearance on the circumference of a cell. In this case, the binarization threshold value is determined to be 1000.

The image analyzing unit 532 separates neighboring cell body masks. The image analyzing unit 532 excludes cell body masks corresponding to one or more cells present on an edge of the quantitative phase image. The image analyzing unit 532 derives roundness of each of one or more remaining cell body masks other than the cell body masks corresponding to the one or more cells present on the edge of the quantitative phase image. The image analyzing unit 532 extracts a cell body mask of which the roundness is equal to or greater than a roundness threshold value on the basis of the derived roundness of each of the one or more cell body masks. An example of the roundness threshold value is 0.8.

The image analyzing unit 532 calculates a cell diameter of each of the extracted one or more cell body masks. For example, the image analyzing unit 532 calculates the cell diameter using cell diameter=$2 \times \sqrt{(\text{area of cell body mask}/\pi)}$.

The image analyzing unit 532 calculates a maximum luminance value in the cell body mask (in a range of the cell body masks) by causing the cell body mask to come into contact with (overlap) an image of a cell included in the quantitative phase image.

The image analyzing unit 532 calculates the refractive index difference $\Delta n$ between the cell and the medium by dividing the maximum luminance value by the cell diameter on the basis of the calculated maximum luminance value in the cell body mask and the calculated cell diameter. The image analyzing unit 532 prepares a histogram with the calculated refractive index difference $\Delta n$ as the horizontal axis and with Count as the vertical axis.

The image analyzing unit 532 determines the living-dead determination threshold value on the basis of the prepared histogram. When the quantitative phase analysis result illustrated in FIG. 11 is acquired, the image analyzing unit 532 determines the living-dead determination threshold value to be the refractive index difference $\Delta n=400$ in the vicinity of a boundary between a ground in which the refractive index differences ($\Delta n$) is large and a group in which the refractive index differences ($\Delta n$) is small. The image analyzing unit 532 counts one or both of living cells and dead cells on the basis of the determined living-dead determination threshold value.

In order to analyze living states of cells, the image analyzing unit 532 derives a survival rate and a living cell density on the basis of the count result of living cells and the count result of dead cells. Specifically, the image analyzing unit 532 may derive the survival rate using Expression (3) on the basis of the count result of living cells.

$$\text{Survival rate [\%]}=100\times(\text{number of living cells})/(\text{number of observed cells})\ldots(3)$$

In the example illustrated in FIG. 11, the survival rate is calculated as 56%.

The image analyzing unit 532 derives the living cell density on the basis of the count result of living cells. For example, the image analyzing unit 532 may derive the living cell density using Expression (4).

$$\text{Living cell density [cells/mL]}=(\text{number of living cells})/(\text{observation field capacity})...\qquad(4)$$

An example of calculation from cells observed in one quantitative phase image has been described above, but the present invention is not limited to this example. For example, a plurality of positions may be imaged while changing an imaging position and a plurality of quantitative phase images may be used. By using a plurality of quantitative phase images, it is possible to count more cells and thus to enhance accuracy of the living cell density or the survival rate.

(Operation of analysis system)

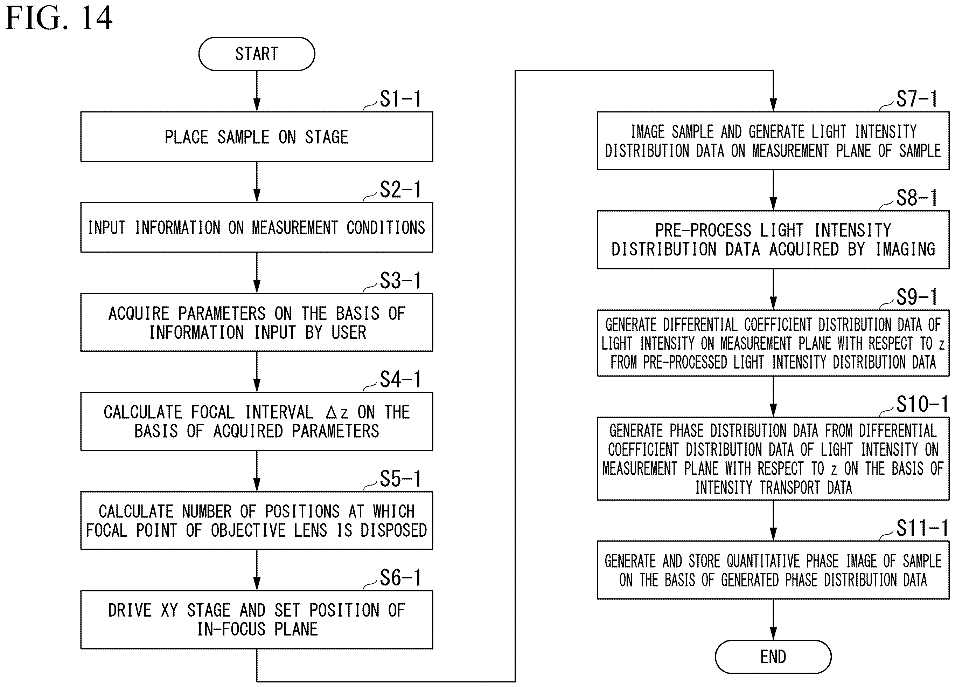

FIG. 14 is a flowchart illustrating Example 1 of an operation flow in the analysis system according to this embodiment. A method of generating a quantitative phase image will be described below with reference to FIG. 14.

(Step S1-1)

In the microscope body 100, a sample is placed on the stage 8. An example of the sample is a plurality of cultured cells.

(Step S2-1)

Information on measurement conditions is input to the input unit 41 of the analysis device 40.

(Step S3-1)

The control unit 50 of the analysis device 40 acquires the information input by a user. The device control unit 51 of the control unit 50 acquires parameters required for imaging the sample S using the microscope body 100 by appropriately calculating the parameters on the basis of the input data input by the user. Examples of the parameters include (I) a wavelength of illumination light, (II) a numerical aperture of an objective lens, (III) a refractive index between the objective lens and the sample, (IV) a lateral magnification of the imaging optical system, (V) a pixel size of the detection unit 9, and (VI) a phase restoration parameter.

(Step S4-1)

The optimal condition calculating unit 511 of the analysis device 40 calculates a focal interval $\Delta z$ on the basis of the acquired parameters.

(Step S5-1)

The optimal condition calculating unit 511 of the analysis device 40 calculates the number of positions at which the focal point of the objective lens is disposed on the basis of the calculated focal interval $\Delta z$.

(Step S6-1)

The device control unit 51 of the analysis device 40 drives the XY stage and sets a position of an in-focus plane.

(Step S7-1)

The image creating unit 52 of the analysis device 40 images the sample on the basis of the (I) to (VI) parameters acquired in Step S3-1, the focal interval and the number of positions at which the focal position of the objective lens is disposed calculated in Step S5-1, and the set position of the in-focus plane and generates light intensity distribution data on the measurement plane of the sample. When the focal point of the objective lens 21a is disposed on the measurement planes i2 and i3, the image creating unit 52 causes the imaging optical system 7 to form an image of the sample S on the detection plane of the detection unit 9 and causes the detection unit 9 to detect light from the sample S.

The detection signals from the pixels of the detection unit 9 corresponding to the cases in which the focal point of the objective lens 21a is disposed on the measurement planes i2 and i3 are input to the control unit 50, and light intensity distribution data in which the positions of the pixels and light intensities based on the detection signals are correlated is generated by the image creating unit 52.

(Step S8-1)

The phase restoration processing unit 521 of the analysis device 40 pre-processes the light intensity distribution data acquired through imaging by generating the light intensity distribution data in which the positions of the pixels of the detection unit 9 and light intensities based on the input detection signals are correlated.

(Step S9-1)

The phase restoration processing unit 521 of the analysis device 40 calculates $dI/dz=(I3-I2)/2\Delta z$ corresponding to the differential coefficient of the light intensity on the measurement plane i1 with respect to z by dividing a difference between intensity values of two points including a point on the measurement plane i2 and a point on the measurement plane i3 and located at corresponding positions such as positions with the same coordinates on the XY plane by $2\Delta z$ which is a distance between the measurement plane i2 and the measurement plane i3.

(Step S10-1)

The phase restoration processing unit 521 of the analysis device 40 generates phase distribution data corresponding to the phase distribution $\phi$ in the sample S including the measurement plane i1 or the like from the generated light intensity distribution data on the basis of the intensity transport equation, the focal interval $\Delta z$ set by the optimal condition calculating unit 511, and the like.

(Step S11-1)

The image constructing unit 522 of the analysis device 40 generates a two-dimensional or three-dimensional quantitative phase image on the basis of the phase distribution data generated by the phase restoration processing unit 521 and stores the generated quantitative phase image in the storage unit 44.

In the flowchart illustrated in FIG. 14, a process of determining the number of captured images may be added before Step S 1-1. For example, the number of captured images may be determined as follows. An observation target was cultured in a culturing liquid volume 800 [mL]. A part of the liquid in a bioreactor was taken and the number of living cells was 5E+7 [cells/mL] using a cell counter. Accordingly, the number of living cells in the bioreactor was estimated to be 4E+10. The number of necessary samples was predicted to be 1844 [cells] with required accuracy 3% and reliability 99%.

On the other hand, since a sample for cell observation has a diameter $\phi$ 15 [mm] and a thickness 0.030 [mm], the number of cells in the sample is calculated as 3.09E+5 [cells/mL] when the cell concentration is 5E+7 [cells/mL]. Since an observation field of view at a zoom ratio 1.5 and a magnification 20 is 0.391 [mm], the number of cells in the field of view is calculated to be 268. Accordingly, the number of captured images is calculated as 1844/268=7, and cell states in the bioreactor can be evaluated by analyzing the cells in the observation field of view at different seven positions.

When the process of determining the number of captured images is added, a process of determining whether the number of captured images has been reached is added after Step S11 -1. The flow ends when it is determined that the number of captured images has been reached, and the flow proceeds to Step S6-1 when it is determined that the number of captured images has not been reached.

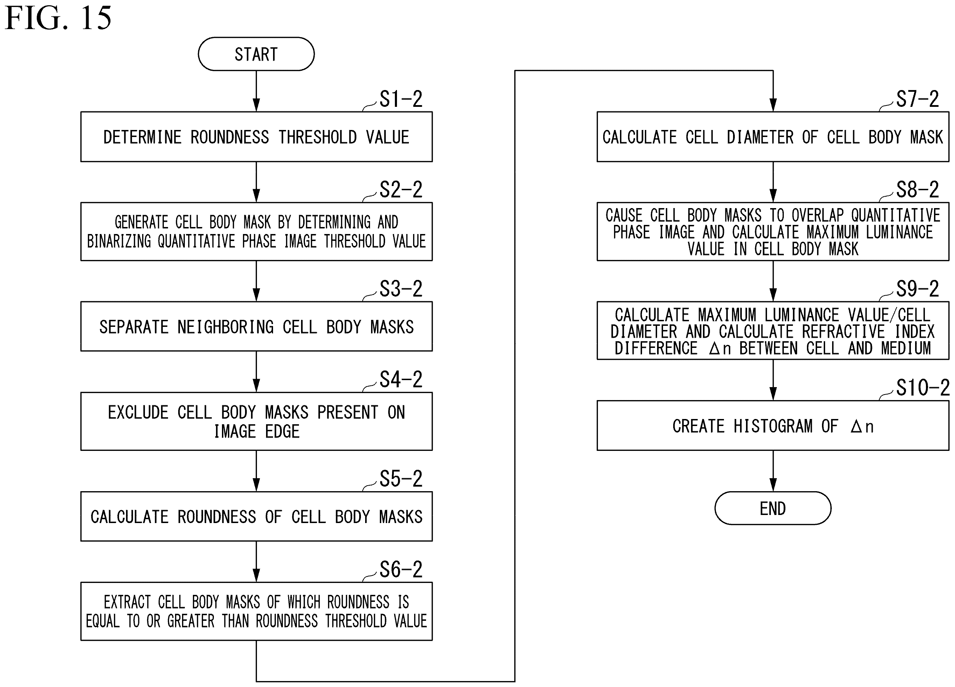

FIG. 15 is a flowchart illustrating Example 2 of the operation flow of the analysis system according to this embodiment. A method of preparing a histogram of a refractive index difference ($\Delta$n) will be described below with reference to FIG. 15.

(Step S1-2)

The image analyzing unit 532 of the analysis device 40 determines a roundness threshold value.

(Step S2-2)

The image analyzing unit 532 of the analysis device 40 determines a quantitative phase image threshold value and generates cell body masks on the basis of a binarization result of the quantitative phase image.

(Step S3-2)

The image analyzing unit 532 of the analysis device 40 separates neighboring cell body masks on the basis of the binarization result of the quantitative phase image.

(Step S4-2)

The image analyzing unit 532 of the analysis device 40 excludes the cell body masks corresponding to one or more cells located on the edge of the quantitative phase image.

(Step S5-2)

The image analyzing unit 532 of the analysis device 40 derives roundness of one or more remaining cell body masks other than the cell body masks corresponding to the one or more cells located on the edge of the quantitative phase image.

(Step S6-2)

The image analyzing unit 532 of the analysis device 40 extracts a cell body mask of which the roundness is equal to or greater than the roundness threshold value on the basis of the derived roundness of each of the one or more cell body masks.

(Step S7-2)

The image analyzing unit 532 of the analysis device 40 calculates a cell diameter of each of the extracted one or more cell body masks.

(Step S8-2)

The image analyzing unit 532 of the analysis device 40 calculates a maximum luminance value in each cell body mask by causing the cell body masks to overlap on the images of the cells included in the quantitative phase image.

(Step S9-2)

The image analyzing unit 532 of the analysis device 40 calculates the refractive index difference Δn between a cell and a medium by dividing the maximum luminance value by the cell diameter on the basis of the calculated maximum luminance value and the calculated cell diameter in the cell body mask.

(Step S10-2)

The image analyzing unit 532 of the analysis device 40 prepares a histogram with the calculated refractive index differences Δn as the horizontal axis and with the Count as the vertical axis.

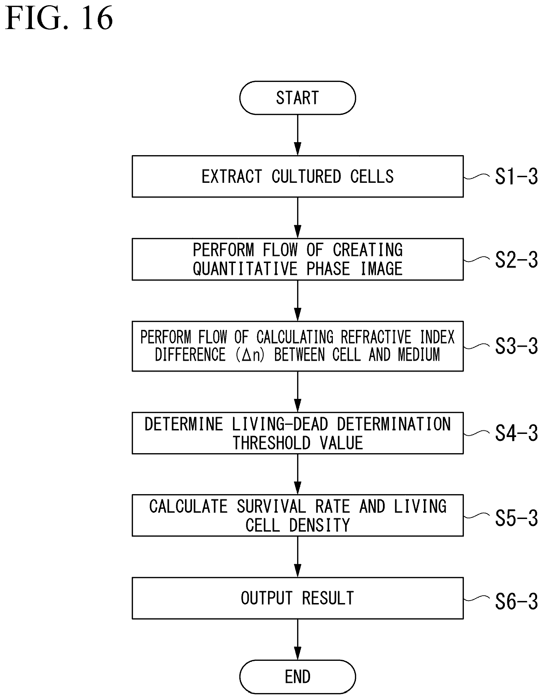

FIG. 16 is a flowchart illustrating Example 3 of the operation flow of the analysis system according to this embodiment. A method of noninvasively calculating a survival rate of a cell will be described below with reference to FIG. 16.

(Step S 1-3)

A cultured cell is extracted. An example of the cultured cell is a plurality of cultured cells.

(Step S2-3)

An operation flow of creating a quantitative phase image is performed. The operation flow of creating a quantitative phase image has been described above with reference to FIG. 14, and thus description thereof will be omitted herein.

(Step S3-2)

An operation flow of calculating a refractive index difference between a cell and a medium is performed. The operation flow of calculating a refractive index difference between a cell and a medium has been described above with reference to FIG. 15, and thus description thereof will be omitted.

(Step S4-3)

The image analyzing unit 532 of the analysis device 40 determines a living-dead determination threshold value on the basis of the prepared histogram.

(Step S5-3)

The image analyzing unit 532 of the analysis device 40 derives a survival rate and a living cell density on the basis of the determined living-dead determination threshold value in order to analyze living states of cells.

(Step S6-3)

The image analyzing unit 532 of the analysis device 40 outputs an analysis result indicating the living states of the cells. Examples of the analysis result include the number of living cells, the number of dead cells, the survival rate, the living cell density [cells/mL], a cell diameter distribution, an average cell diameter, a maximum cell diameter, a minimum cell diameter, a roundness distribution, an average roundness, a maximum roundness, and a minimum roundness. The image analyzing unit 532 derives the number of living cells, the number of dead cells, the survival rate, the living cell density, the cell diameter distribution, the average cell diameter, the maximum cell diameter, the minimum cell diameter, the roundness distribution, the average roundness, the maximum roundness, and the minimum roundness and outputs derivation results of the number of living cells, the number of dead cells, the survival rate, the living cell density, the cell diameter distribution, the average cell diameter, the maximum cell diameter, the minimum cell diameter, the roundness distribution, the average roundness, the maximum roundness, and the minimum roundness.
In the aforementioned embodiment, an example in which the image analyzing unit 532 calculates a maximum luminance value in a cell body mask (a range of a cell body mask) by causing the cell body mask to come into contact with (to overlap) an image of a cell included in the quantitative phase image has been described above, but the present invention is not limited to this example. For example, the image analyzing unit 532 may calculate an integrated value of the luminance value in the cell body mask by causing the cell body mask to come into contact with (to overlap) an image of a cell included in the quantitative phase image. Thereafter, the operation flow is performed using the integrated value of the luminance value instead of the maximum luminance value.

In the aforementioned embodiment, an example using a method based on the intensity transport equation has been described above as the method of creating a quantitative phase image, but the present invention is not limited to this example. For example, a method using interference may be used as the method of creating a quantitative phase image. By using the method based on the intensity transport equation, the present invention can be applied to an amplitude object (a cell stained in an opaque state) in addition to the phase object on which phase unwrapping does not need to be performed in comparison with the interference method.

In the aforementioned embodiment, determination of life or death of a cell may be performed noninvasively in a time series. The image analyzing unit 532 of the analysis device 40 may also display information indicating change of a shape of cells or the number of cells in a culturing period including a change with time of the survival rate in addition to the aforementioned analysis results.

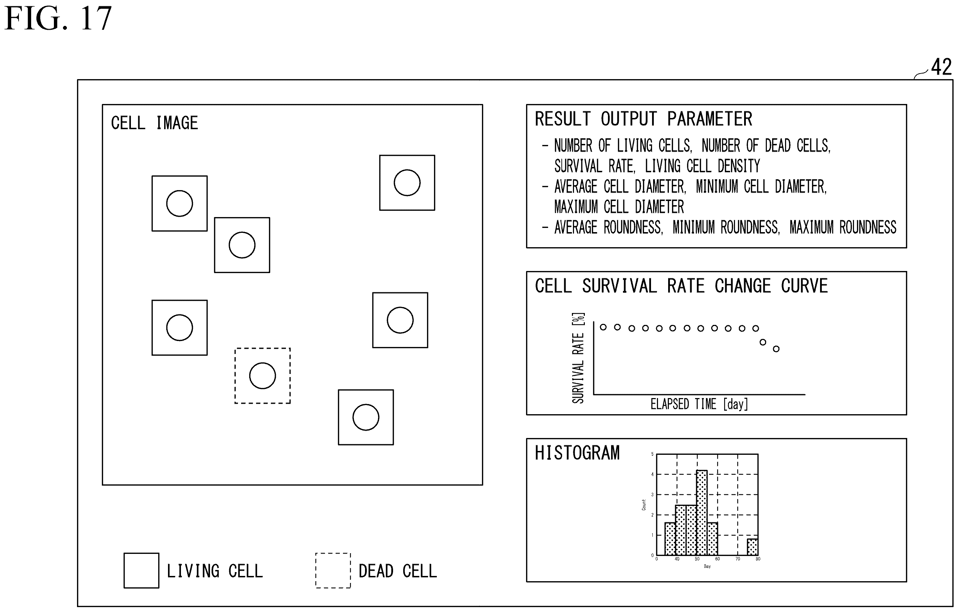

FIG. 17 is a conceptual diagram illustrating an example of an analysis result displayed by the analysis device included in the analysis system according to this embodiment. As illustrated in FIG. 17, the display unit 42 displays cell images, result output parameters (analysis result), a cell survival rate change curve, and a histogram.

In the aforementioned embodiment, the image analyzing unit 532 may search for a predetermined cell on the basis of the refractive index differences Δn. Since an activation state of a cell can be determined on the basis of the refractive index differences Δn, the image analyzing unit 532 can consider a group in which the refractive index differences Δn is high as a superior cell line. For example, the image analyzing unit 532 may search for a superior cell line, and application to cell line development is also possible.

With the analysis system 1 according to this embodiment, the analysis system 1 includes the acquisition unit 531 configured to acquire a cell image including images of a plurality of cultured cells and the image analyzing unit 532 configured to derive a refractive index difference between the plurality of cultured cells included in the cell image and the medium and to drive one or both of a survival rate of the plurality of cultured cells and a living cell density of living cells in the cultured cells on the basis of refractive index difference information for identifying the refractive index difference.

With this configuration, it is possible to observe cells in a living state achromatically and noninvasively in real time (observation equivalent to fluorescent stain is possible). Individual cells can be segmented. It is possible to quantitatively analyze morphology of cells (thickness, surface area, volume, and the like). It is possible to noninvasively evaluate dead cells inline.

In the analysis system 1, the acquisition unit 531 acquires a quantitative phase image on the basis of light intensity distribution data of the cell image, and the image analyzing unit 532 derives information of the refractive index difference between the plurality of cultured cells included in the quantitative phase image and the medium on the basis of cell luminance values in the quantitative phase image and the cell diameters of the cultured cells.

With this configuration, the image analyzing unit 532 can derive the refractive index difference information between the plurality of cultured cells included in the quantitative phase image and the medium on the basis of cell luminance values in the quantitative phase image and the cell diameters of the cultured cells. It is possible to noninvasively evaluate the survival rate and the living cell density inline.

In the analysis system 1, the image analyzing unit 532 derives the refractive index difference information on the basis of Expression (1):

$$\text{Phase value} = (2 \times \pi \times \Delta n \times \text{cell thickness})/\lambda \qquad \dots(1)$$

In Expression (1), $\lambda$ is an observation wavelength, Δn is a refractive index difference between a cell and a medium, a phase value corresponds to the cell luminance value of the quantitative phase image, and the cell thickness corresponds to the cell diameter. The refractive index difference information is acquired by dividing the cell luminance value by the cell diameter.

With this configuration, the image analyzing unit 532 can derive the phase value and thus derive the refractive index difference information on the basis of the derived phase value. It is possible to noninvasively evaluate the survival rate and the living cell density inline. In the analysis system 1, the image analyzing unit 532 determines living cells and dead cells in the cultured cells on the basis of refractive index difference information and derives the survival rate of the cultured cells using Expression (2) on the basis of a count result of the living cells and the number of observed cells:

$$\text{Survival rate [\%]} = 100 \times (\text{number of living cells})/(\text{number of observed cells})$$

$$\dots(2).$$

With this configuration, the image analyzing unit 532 can derive the survival rate on the basis of the count result of the living cells and the number of observed cells. It is possible to noninvasively evaluate the survival rate and the living cell density inline.

In the analysis system 1, the image analyzing unit 532 determines living cells and dead cells in the cultured cells on the basis of the refractive index difference information and derives the living cell density of the living cells using Expression (3) on the basis of a counting result of the living cells and a capacity of a container in an observation field of view:

$$\text{Living cell density [cells/mL]} = \text{(number of living cells)/(observation field capacity)} \qquad (3).$$

With this configuration, the image analyzing unit 532 can derive the living cell density on the basis of the count result of the living cells and the cell volume of the container in the observation field of view. It is possible to noninvasively evaluate the living cell density inline.

(Modified examples of embodiment)

The configuration illustrated in FIG. 1 can be applied to an analysis system 1a according to a modified example of the embodiment. Here, an analysis device 40a is provided instead of the analysis device 40. The analysis system 1a includes a microscope body 100 and an analysis device 40a.

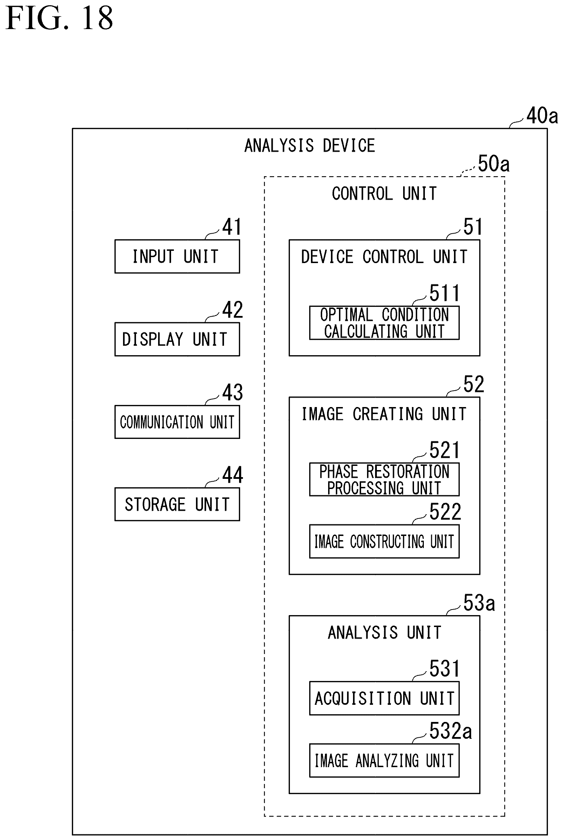

FIG. 18 is a diagram illustrating a configuration of the analysis device included in the analysis system according to the modified example of the embodiment. The analysis device 40a includes an input unit 41, a display unit 42, a communication unit 43, a storage unit 44, and a control unit 50a. The control unit 50a includes a device control unit 51, an image creating unit 52, and an analysis unit 53a. The device control unit 51 includes an optimal condition calculating unit 511. The image creating unit 52 includes a phase restoration processing unit 521 and an image constructing unit 522. The analysis unit 53a includes an acquisition unit 531 and an image analyzing unit 532a.

In the modified example of the embodiment, the analysis device 40a evaluates the refractive index differences (Δn) actually using antibody forming cells and performs prediction of a specific antibody production rate during culturing, monitoring of cells in the culturing process, control thereof, and the like on the basis of a correlation between the specific antibody production rate in the cell culturing process and an average value in a histogram of the refractive index differences (Δn). The histogram of the refractive index differences (Δn) is an example of an index indicating that the cultured cells produce an antibody.

In the modified example of the embodiment, cultured cells are extracted, and an antibody concentration produced by the extracted cultured cells through a biochemical test is calculated. The analysis device 40a calculates a specific antibody production rate of antibody forming cells on the basis of the calculated concentration of antibody products and the living cell density of living cells. The analysis device 40a calculates the living cell density on the basis of refractive index difference information associated with a refractive index difference between a plurality of cells and a medium.

Estimation of a specific antibody production rate of antibody forming cells will be described below. The embodiment can be applied to the microscope body 100 and an observation container. As can be seen from FIG. 11 mentioned in the embodiment, a distribution of the refractive index differences (Δn) in a group of living cells is present in a histogram distribution of the refractive index differences (Δn). Protein or amino acid which is a constituent element thereof has a higher molar refractive index than water which is main component of the medium. When an amount of protein or amino acid which is a constituent element thereof in a cell is large, it can be considered that the refractive index difference (Δn) increases and the cell is activated, and the molar refractive index is predicted to serve as an index of an antibody production capacity.

In the modified example of the embodiment, transfection is performed on cells and evaluation was performed using CHO cells improved to generate an antibody (IgG).

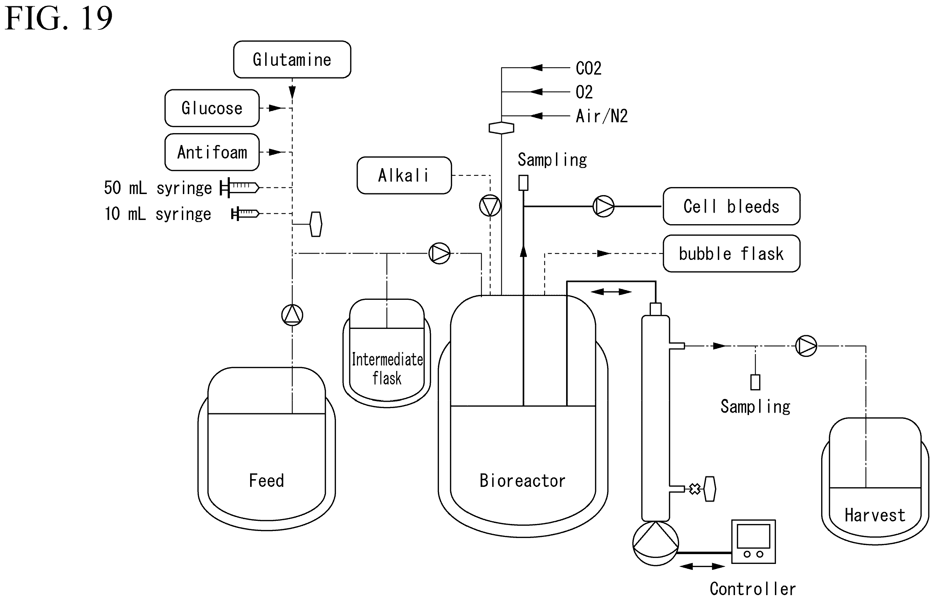

FIG. 19 is a diagram illustrating an example of a culturing device.

Cells cultured to about 0.5E+6 [cells/mL] in a flask were transferred to a bioreactor and were cultured. This culturing device is called a perfusion culturing device and is designed to appropriately add a medium including glutamine and glucose which are nutrients of a cell in addition to a function of removing bubbles through culturing, a function of uniformly agitating the bioreactor, a function of uniformizing the temperature in the bioreactor, and a function of monitoring dissolved oxygen or pH. Accordingly, in order to maintain a constant amount of liquid in the bioreactor, a medium (harvest) including antibodies other than cells is recovered under the control of a controller.

In order to maintain a constant number of living cells in the bioreactor, thinning of cells was appropriately performed (cell bleed (cell bleeds)). In a period from start to end of culturing, an antibody concentration in the bioreactor and an antibody concentration in the harvest were measured using a biochemical test method at predetermined timings. A specific antibody production rate [pg/cell/day] which is an index indicating how much an antibody forming cell produces antibodies per day was calculated by Expression (5).

[Math. 1]

$$\text{Specific antibody production rate} \left[\frac{\text{pg}}{\text{cell} \cdot \text{day}}\right] = \frac{\text{Antibody product concentration in culturing days}}{\int_{t1}^{t2} Xdt} \quad \cdots (5)$$

A trapezoidal rule was used to calculate an approximate value of an integral which is a denominator of Expression (5) as expressed by Expression (6).

[Math. 2]

$$\int_{t1}^{t2} Xdt \sim \frac{(t2-t1) \times (Xt1+Xt2)}{2} \quad \cdots (6)$$

In Expression (5) and Expression (6), ti (where i=1, 2) is a culturing day, and Xti is a living cell density on the culturing day ti. The antibody concentrations of the bioreactor and the harvest were measured at predetermined timings, a weighted sum of perfusion rates (return rates to the bioreactor in a process directed to the harvest) was calculated, and the specific antibody production rate was determined on the basis of Expression (5) and Expression (6). The living cell density was calculated using the method described above in the embodiment.

At the time of measuring an antibody production concentration in the bioreactor, a part of a cell suspension was injected by 5.3 $\mu$L into the container described above with reference to FIG. 8A and the container was placed on the stage 8 of the microscope body 100. Bright-field observation was performed using the microscope body 100 and using a 20x objective lens (NA 0.45), a zoom magnification 1.5x, and a white light emitting diode (LED) light source as a transmission illumination. Culturing was performed under the conditions in which the living cell density in the bioreactor (800 mL) is 50E+6 [cells/mL]. 1844 living cells can be observed for evaluation with required accuracy of 3% and reliability of 99% in observing the total number of living cells in the bioreactor.

When the container illustrated in FIG. 8A is used, the number of cells in the observation field of view of 0.391 mm of the 20x objective lens is calculated to be 268, and the number of samples enough to evaluate cells with the living cell density 50E+6 [cells/mL] in the bioreactor 800 mL is obtained by performing imaging at seven different positions when it is intended to observe 1844 cells.

Therefore, the stage 8 of the microscope body 100 was moved, and bright-field images at a focal position and positions shifted 5 $\mu$m forward and backward from the focal position were acquired at seven different positions. A quantitative phase image was constructed for the acquired bright-field images using the method described above with reference to FIG. 14. The refractive index difference ($\Delta$n) in the quantitative phase image was derived using the method described above with reference to FIG. 15. The living cell density in the quantitative phase image was derived using the method described above with reference to FIG. 16. These processes were repeated in a designated culturing period.

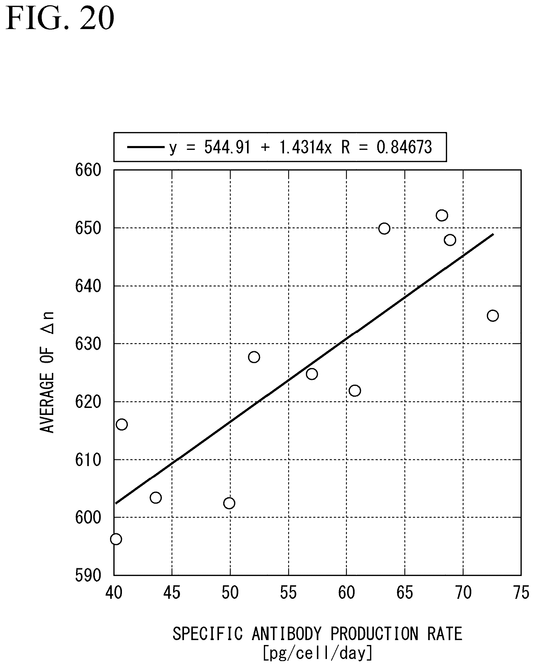

FIG. 20 is a diagram illustrating an example of a relationship between a specific antibody production rate and an average value of the refractive index differences ($\Delta$n). FIG. 20 illustrates a relationship between the specific antibody production rate and refractive index difference information such as an average value of the refractive index differences ($\Delta$n). As illustrated in FIG. 20, there is an interesting correlation between the specific antibody production rate and the average value of the refractive index differences ($\Delta$n). In FIG. 20, there is a correlation between the specific antibody production rate and the average value of the refractive index differences ($\Delta$n) at a correlation coefficient of about 0.85.

As described above, an analytical curve indicating the correlation between the specific antibody production rate and the refractive index difference information can be determined using the specific antibody production rate and the average value of the refractive index differences ($\Delta$n) by acquiring an antibody product concentration in advance at the time of determination of culturing conditions and performing construction of a quantitative phase image and calculation of a distribution of the refractive index differences ($\Delta$n) at that time. This represents that the specific antibody production rate in the bioreactor can be predicted by calculating the average value of the refractive index differences ($\Delta$n) from the quantitative phase image.

The image analyzing unit 532 can be used as the image analyzing unit 532a. The image analyzing unit 532a acquires a quantitative phase image from the acquisition unit 531. The image analyzing unit 532a determines a quantitative phase image threshold value and binarizes the quantitative phase image on the basis of the determined quantitative phase image threshold value.

The image analyzing unit 532a generates cell body masks on the basis of the binarization result of the quantitative phase image.

The image analyzing unit 532a separates neighboring cell body masks. The image analyzing unit 532a excludes cell body masks corresponding to one or more cells present on an edge of the quantitative phase image. The image analyzing unit 532a derives roundness of each of one or more remaining cell body masks other than the cell body masks corresponding to the one or more cells present on the edge of the quantitative phase image. The image analyzing unit 532a extracts a cell body mask of which the roundness is equal to or greater than a roundness threshold value on the basis of the derived roundness of each of the one or more cell body masks. An example of the roundness threshold value is 0.8.

The image analyzing unit 532a calculates a cell diameter of each of the extracted one or more cell body masks. For example, the image analyzing unit 532a calculates the cell diameter using cell diameter=$2\times\sqrt{}$(area of cell body mask/$\pi$).

The image analyzing unit 532a calculates a maximum luminance value in each cell body mask by causing the cell body mask to come into contact with (overlap) an image of a cell included in the quantitative phase image.

The image analyzing unit 532a calculates the refractive index difference $\Delta$n between a cell and a medium by dividing the maximum luminance value by the cell diameter on the basis of the calculated maximum luminance value in each cell body mask and the calculated cell diameter. The image analyzing unit 532a prepares a histogram with the calculated refractive index difference $\Delta$n as the horizontal axis and with Count as the vertical axis.

The image analyzing unit 532a derives the living-dead determination threshold value on the basis of the prepared histogram. The image analyzing unit 532a acquires the number of living cells on the basis of the derived living-dead determination threshold value. The image analyzing unit 532a calculates the specific antibody production rate on the basis of the acquired number of living cells. The image analyzing unit 532a calculates the average value of the refractive index differences $\Delta$n on the basis of the calculation result of the refractive index difference $\Delta$n between a cell and a medium. The image analyzing unit 532a plots a correlation between the specific antibody production rate and the average value of the refractive index differences $\Delta$n on the basis of the calculation result of the specific antibody production rate and the calculation result of the average value of the refractive index differences ($\Delta$n). The image analyzing unit 532a stores a specific antibody production rate analytical curve which is a plotting result of the correlation between the specific antibody production rate and the average value of the refractive index differences $\Delta$n in the storage unit 44.

The image analyzing unit 532a reads the specific antibody production rate analytical curve stored in the storage unit 44. The image analyzing unit 532a creates a quantitative phase image of the cultured cells. The image analyzing unit 532a calculates the average value of the refractive index differences $\Delta$n between a cell and a medium on the basis of the created quantitative phase image of the cultured cells. The image analyzing unit 532a calculates a specific antibody production on the basis of the specific antibody production rate analytical curve and the calculation result of the average value of the refractive index differences ($\Delta$n).

(Operation of analysis system)

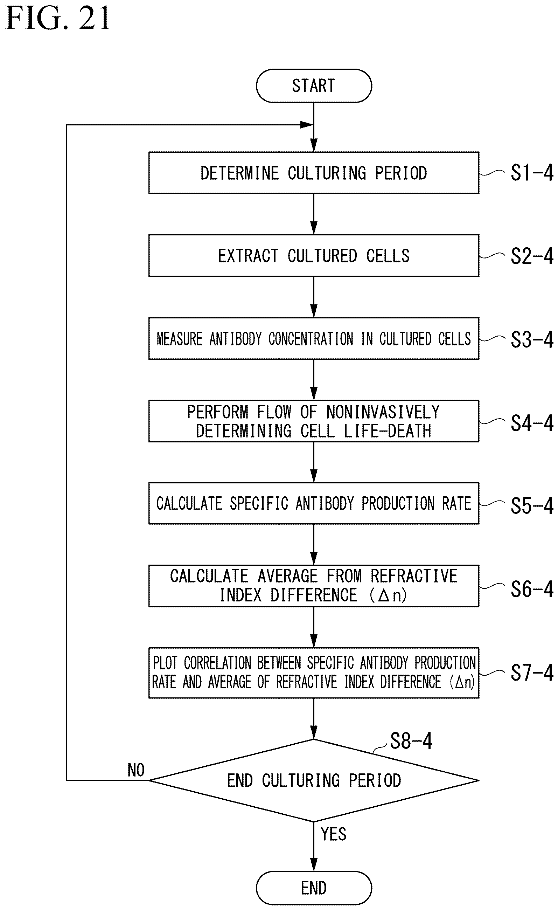

FIG. 21 is a flowchart illustrating an example of an operation flow in the analysis system according to the modified example of the embodiment. A method of preparing a specific antibody production rate analytical curve will be described below with reference to FIG. 21.

(Step S1-4)

A culturing period is determined.

(Step S2-4)

Cultured cells are extracted.

(Step S3-4)

An antibody concentration in the extracted cultured cells is measured. A concentration of antibody products produced by the cultured cells is calculated.

(Step S4-4)

An operation flow of noninvasively determining life and death of a cell is performed. The operation flow of noninvasively determining life and death of a cell has been described above with reference to FIG. 16, and thus description thereof will be omitted herein.

(Step S5-4)

The image analyzing unit 532a of the analysis device 40a calculates a specific antibody production rate. The image analyzing unit 532a calculates the specific antibody production rate of an antibody forming cell on the basis of the antibody product concentration and the living cell density of living cells.

(Step S6-4)

The image analyzing unit 532a of the analysis device 40a calculates refractive index information such as the average value of the refractive index differences ($\Delta$n) from the refractive index differences ($\Delta$n) prepared in the operation flow of noninvasively determining life and death of a cell.

(Step S7-4)

The image analyzing unit 532a of the analysis device 40a plots a correlation between the calculated specific antibody production rate and the calculated average value of the refractive index differences ($\Delta$n). The image analyzing unit 532a stores the plotting result of the correlation between the specific antibody production rate and the average value of the refractive index differences ($\Delta$n) in the storage unit 44.

(Step S8-4)

The image analyzing unit 532a of the analysis device 40a determines whether the culturing period set in Step S1-4 has expired. When the culturing period has not expired, the operation flow returns to Step S 1-4. When the culturing period has expired, the operation flow ends.

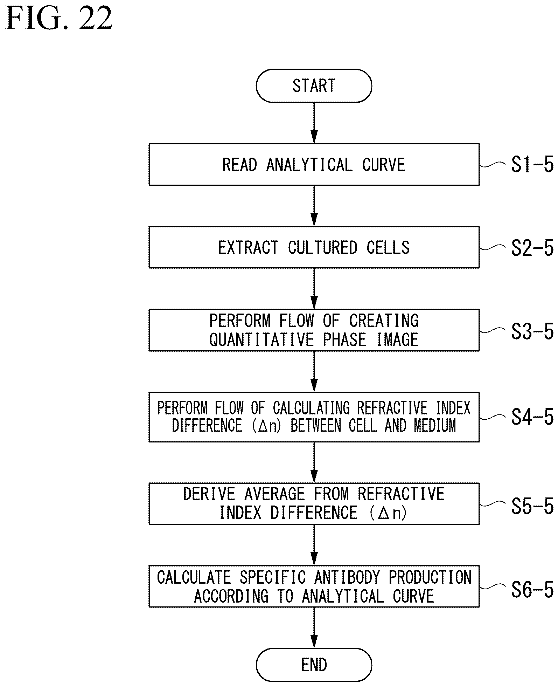

FIG. 22 is a flowchart illustrating an example of an operation flow in the analysis system according to the modified example of the embodiment. A method of calculating a specific antibody production will be described below with reference to FIG. 22. Here, it is assumed that a specific antibody production rate analytical curve is stored in the storage unit 44.

(Step S1-5)

The image analyzing unit 532a of the analysis device 40a reads the specific antibody production rate analytical curve stored in the storage unit 44.

(Step S2-5)

Cultured cells are extracted.

(Step S3-5)

An operation flow of creating a quantitative phase image is performed. The operation flow of creating a quantitative phase image has been described above with reference to FIG. 14, and thus description thereof will be omitted.

(Step S4-5)

An operation flow of calculating a refractive index difference between a cell and a medium is performed. The operation flow of calculating a refractive index difference between a cell and a medium has been described above with reference to FIG. 15, and thus description thereof will be omitted.

(Step S5-5)

The image analyzing unit 532a of the analysis device 40a calculates an average value of the refractive index differences ($\Delta$n) on the basis of the calculation result of the refractive index difference ($\Delta$n) between a cell and a medium.

(Step S6-5)

The image analyzing unit 532a of the analysis device 40a calculates a specific antibody production on the basis of the specific antibody production rate analytical curve and the calculation result of the average value of the refractive

index differences (∆n).

In the modified example of the embodiment, the image analyzing unit 532a may acquire a median value of the refractive index differences ∆n from a histogram with the refractive index differences ∆n as the horizontal axis and with Count as the vertical axis and derive the specific antibody production rate on the basis of the acquired median value and a plotting result of a correlation between the calculation result of the median value and the antibody production rate.

In the modified example of the embodiment, cell culturing conditions may be set on the basis of the analysis result from the image analyzing unit 532a.

In a therapeutic antibody producing process using antibody forming cells, cells are cultured in a long term using the culturing device illustrated in FIG. 19, and antibodies produced during culturing are extracted. When the culturing period progresses and a culturing end stage comes, activation capability of antibodies in the bioreactor vanishes. As a result, a decrease in specific antibody production rate or a decrease in survival rate occurs, but the decrease in specific antibody production rate is more remarkable than the decrease in survival rate. Therefore, the image analyzing unit 532a may monitor the specific antibody production rate, end the culturing period or derive the culturing conditions on the basis of behavior thereof, and cause the control unit of the culturing device to end the culturing period or to change the culturing conditions.

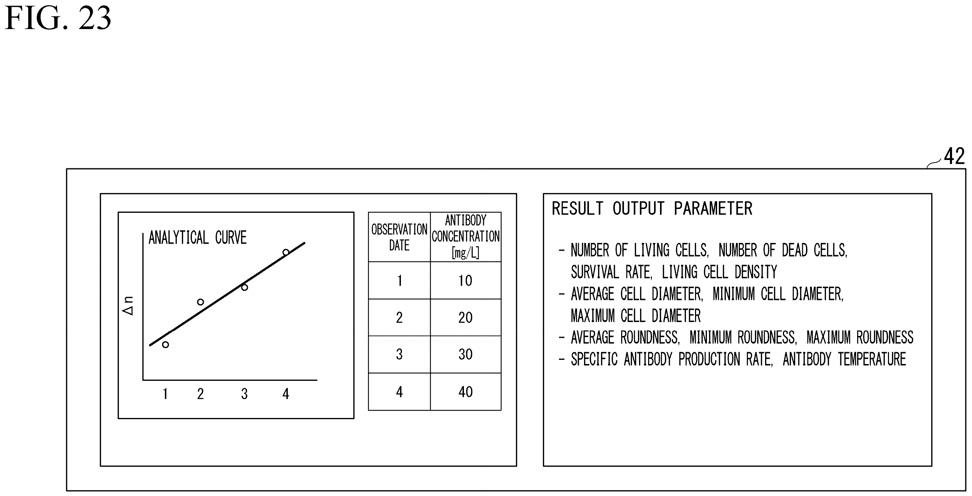

FIG. 23 is a conceptual diagram illustrating Example 1 of a display screen which is displayed by the analysis device included in the analysis system according to the modified example of the embodiment. As illustrated in FIG. 23, the display unit 42 displays a cell image, an analytical curve, and result output parameters (analysis results).

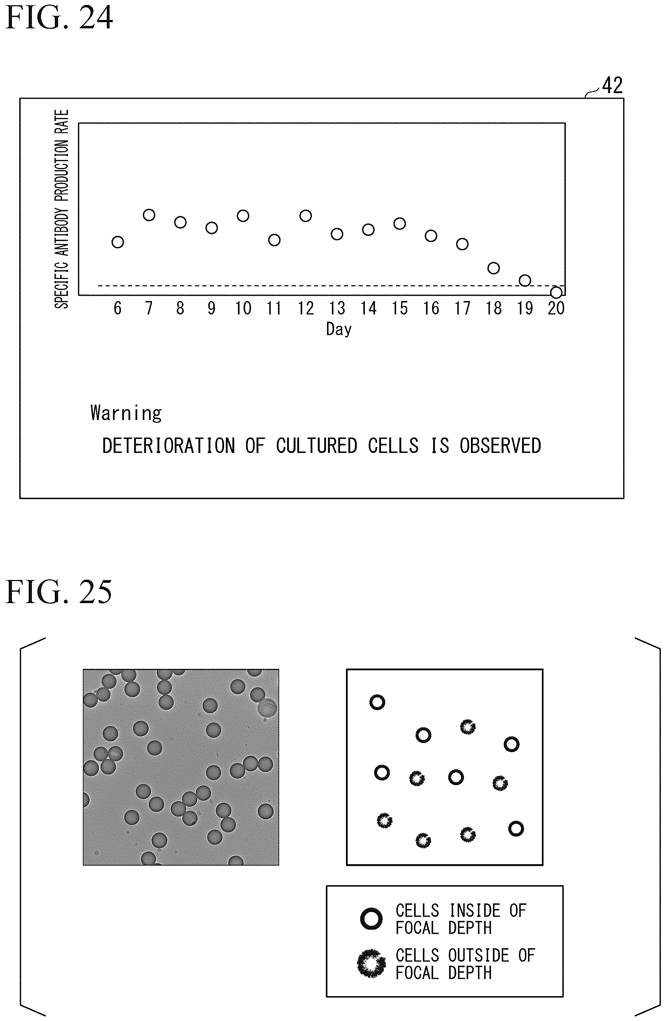

FIG. 24 is a conceptual diagram illustrating Example 2 of the display screen which is displayed by the analysis device included in the analysis system according to the modified example of the embodiment. As illustrated in FIG. 24, the display unit 42 displays a change of the specific antibody production rate with time. The image analyzing unit 532a may issue a warning when the specific antibody production rate is equal to or less than the specific antibody production rate threshold value (indicated by a dotted line) on the basis of the change of the specific antibody production rate with time.

In the embodiment and the modified example of the embodiment, a cell line established already is used. In the therapeutic antibody producing process, a process of constructing an antibody forming cell line is present as an upstream process thereof and is the most important process in determining an antibody production capacity. Gene recombination on a host cell has to be performed, and a cell line with excellent proliferation and excellent antibody productivity has to be found in uncountable diversity. As described above, a luminance value of a cell in a quantitative phase image indicates information of a cell thickness and a refractive index difference between a cell and a medium. The refractive index difference (∆n) between a cell and a medium acquired by dividing the luminance value by the cell thickness can be considered to indicate an activation state of a cell.

Therefore, cells with a large refractive index difference (∆n) may be extracted from the cells in the quantitative phase image, the extracted cells may be isolated, and proliferation may be checked. With this configuration, it is possible to select a cell line with excellent antibody productivity and excellent proliferation. In addition to search for a cell line with an excellent evaluation result in one cell, it is possible to evaluate chemical resistance of bacteria, for example, using a system in which bacteria are enclosed in liquid drops and to determine what culturing condition is excellent for the bacteria on the basis of the quantitative phase image by setting various culturing conditions in the liquid droplets.

According to the modified example of the embodiment, the image analyzing unit 532a of the analysis system 1a calculates a specific antibody production rate of antibody forming cells which is an index at which the cultured cells produce antibodies on the basis of a concentration of an antibody product produced by the cultured cells and a living cell density of the living cells using the concentration of the antibody product as an input value.

With this configuration, the analysis system 1a can calculate the specific antibody production rate of the antibody forming cells on the basis of the concentration of an antibody product produced by the cultured cells and the living cell density of the living cells. At the time of determination of living and dead cells, it is possible to predict the antibody production rate without measuring the antibody concentration in the bioreactor through a biochemical test.

In the analysis system 1, the image analyzing unit 532a calculates the antibody production of the antibody forming cells on the basis of a correlation between the refractive index difference information and the specific antibody production rate.

With this configuration, the image analyzing unit 532a can calculate the living cell density on the basis of the histogram of refractive index difference information associated with the refractive index difference between a plurality of cells and a medium.

With this configuration, it is possible to calculate an antibody production on the basis of the correlation between the specific antibody production rate and the refractive index difference information.

By observing cell states in the bioreactor to detect, for example, change of a distribution of refractive index differences ∆n, it is possible to perform determination of end of a culturing period or derivation of culturing conditions such as a glucose concentration. Accordingly, the controller of the culturing device can end the culturing period or change the culturing conditions.

In the analysis system 1, the image analyzing unit 532a prepares a histogram with the refractive index difference as the horizontal axis and with the count as the vertical axis and determines the living cells and the dead cells.

With this configuration, the image analyzing unit 532a can determine the living cells and the dead cells on the basis of the histogram of refractive index difference information associated with the refractive index difference between a plurality of cells and a medium.

In the analysis system 1, the image analyzing unit 532a determines an analytical curve indicating a correlation between the specific antibody production rate and the refractive index difference information and calculates an antibody production on the basis of the determined analytical curve.

With this configuration, since an analytical curve indicating the correlation between the specific antibody production rate and the refractive index difference information can be determined, it is possible to calculate an antibody production on the basis of the determined analytical curve.

(Description of various observation containers)

An improved example of the thin-layer observation container used to observe cells and described above with reference to FIGS. 8A and 8B will be described below.

An observation container (referred to as a thin-layer cell) is an observation container which includes an accommodation unit (an inner space of the container) in which a cell suspension (a medium and a plurality of cells) is accommodated and which is suitable for acquiring and observing a quantitative phase image of a plurality of cells. The accommodation unit is formed such that the thickness of the accommodation unit in the optical-axis direction of the objective lens 21a (the observation optical system) satisfies conditions in which cells floating in the medium stay in an in-focus range (a focal depth range) of the objective lens 21a for a predetermined observation time. The predetermined observation time is an example of an imaging time required for acquiring a quantitative phase image of a plurality of cells.

In the related art, since a hemocytometer or a cell counter observes cells using an observation system with a low magnification, it is not necessary to strictly determine specifications of the container thickness into which a cell suspension is injected. In the embodiment or the like, there is need for observing cell morphology information of floating cells with high accuracy.

In order to accurately measure the cell morphology information of floating cells, the thickness in the optical-axis direction of the objective lens 21a (the observation optical system) of the accommodation unit of the observation container in which a cell suspension is enclosed has to be strictly managed. This is because when the thickness of the accommodation unit is sufficiently larger than the cell diameter (size) of the floating cells (that is, when the cell suspension layer is thick), the positional relationship between cells is not arranged in a direction perpendicular to the optical-axis direction of the objective lens 21a (in the thickness direction of the accommodation unit), and cells stay at positions with different focal positions in the observation field of view. In this case, it is necessary to drive autofocus (AF) for individual cells at the time of observation of the cells and the throughput decreases greatly.

On the other hand, when the thickness of the accommodation unit is small, positions of cells are limited to the direction (a local area) perpendicular to the optical-axis direction of the objective lens 21a, and thus the positional relationship between cells is automatically arranged at positions with almost the same focal position in the observation field of view. The observation container is a thin-layer container for finely image-analyzing the cell morphology information of floating cells.

A method of arranging positions of a plurality of cells in the accommodation unit at positions with almost the same focal position in the observation field of view regardless of conditions of the thickness of the accommodation unit is as follows. By causing an injected cell suspension to flow in the accommodation unit in which a flow channel is formed at a predetermined speed, cells can be gathered in a central part of the accommodation unit with friction (surface tension) of the inner wall forming the accommodation unit, and the cells can be arranged at the center position in the thickness direction of the accommodation unit. This is a method of maximizing a flow rate in the central part of the flow channel and arranging the positions of cells in the flow channel by satisfying so-called laminar flow conditions.

FIG. 25 is a diagram illustrating an example of a bright-field image. In FIG. 25, the left part is a photograph of a bright-field image obtained using a thin-layer container with a thickness of 30 $\mu$m (two times the cell diameter) in which polystyrene beads with a diameter ($\Phi$) of 15 $\mu$m are dispersed in an aqueous solution. Observation conditions with a microscope include an objective lens magnification 40x (a numerical aperture NA of 0.95) and a zoom ratio 1.5x. It can be seen from the left part that all beads in the field of view are located in the focal depth.

On the other hand, the right part is a schematic diagram illustrating an image obtained by dispensing a cell suspension including cells with a diameter ($\phi$) of 10 $\mu$m into a thin-layer container with a thickness of 30 $\mu$m (three times the cell diameter) and observing the thin-layer container. In the right part, cells in the focal depth and cells outside of the focal depth were observed in half-and-half probabilities. As can be seen from this point, it is necessary to adjust the thickness of the accommodation unit in the thin-layer container to be equal to or less than three times the cell diameter or preferably two times the cell diameter.

With this configuration, the thickness of the accommodation unit can satisfy conditions in which cells floating in a medium stay in an in-focus range (in the focal depth range) of the objective lens 21a for a predetermined observation time.

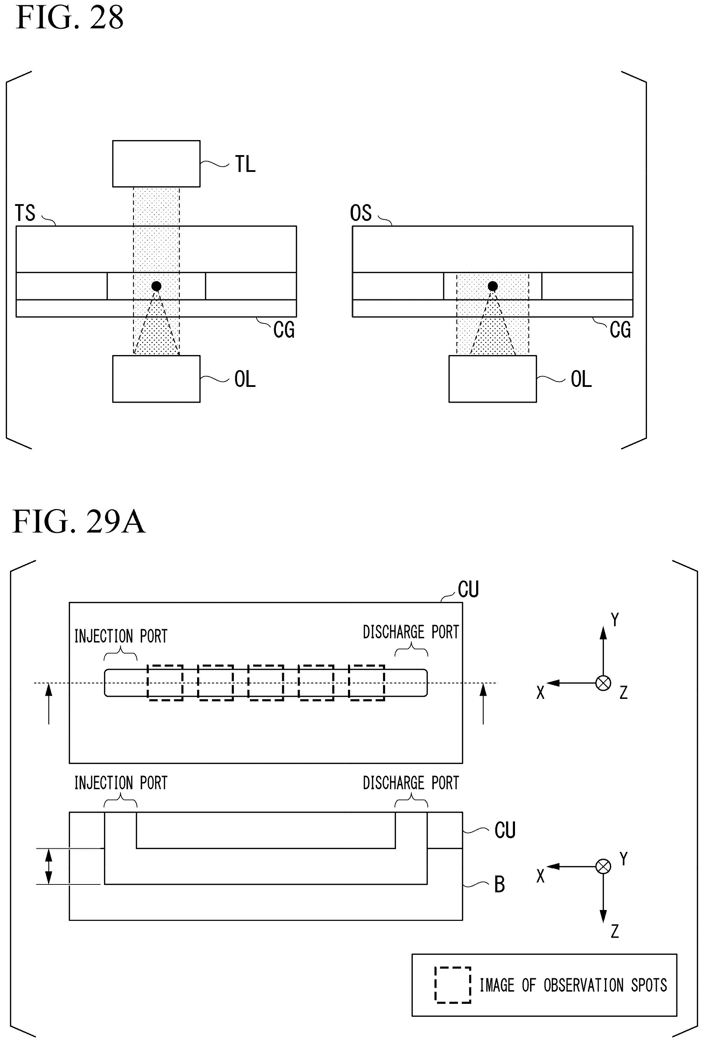

For example, even when the thickness of the accommodation unit in the optical-axis direction of the objective lens 21a is equal to or greater than three times the cell diameter, it is possible to achieve the purpose by forming the accommodation unit as a flow channel as described above. When the accommodation unit is formed as a flow channels as will be described later (in examples subsequent to FIG. 29A), the cells floating in the medium can be arranged in the direction perpendicular to the thickness direction of the accommodation unit and can be made to stay in the focal depth in the optical-axis direction including the focal position of the objective lens 21a for a predetermined observation time.

With this configuration of the accommodation unit, it is possible to stably observe cells by adjusting the focal position of the objective lens 21a with respect to the substrate by a predetermined offset. For example, an autofocus control method using an offset lens described in Japanese Unexamined Patent Application, First Publication No. 2007-148159 can be used as the method of controlling the focal position. With this method, the focal position of the objective lens can be moved to a predetermined position in the accommodation unit using the offset lens after the focal position has been temporarily set to a surface of a cover glass or a slide glass. Accordingly, the focal position of the objective lens can be normally maintained at a predetermined position in the accommodation unit.

The accommodation unit may have an inner surface curved to narrow when seen in the direction perpendicular to the optical-axis direction. Curved mentioned herein means that a flow rate in the flow channel is changed to arrange cells of the accommodation unit in an in-focus range by characterizing the shape of the flow channel. With this configuration, it is possible to increase the number of cells staying in the in-focus range.

A cell concentration dispensed to the thin-layer container is preferably equal to or less than Expression (7).

$$\text{Cell concentration [cells/L]}=0.5\times(W/D)\times(H/D)/(T\times W\times H) \quad …(7)$$

In Expression (7), the accommodation unit of the thin-layer container has a drop area $W\times H$ of a cell suspension and a thickness T [mm]. W denotes a lateral width [mm], H denotes a longitudinal width [mm], and T denotes a thickness. D denotes a cell diameter [mm]. The cell concentration is preferably equal to or less than 50%. Since the cell concentration is equal to or less than 50%, the right side of the expression is multiplied by 0.5. When the cell concentration is excessively high, the cell positions in the optical-axis direction of the observation optical system are deviated.

For example, when the drop area $W\times H$ of the accommodation unit of the thin-layer container is $10\times10$ mm, the thickness T is 0.01 mm, and the cell diameter D is 10 $\mu$m, an upper limit of the cell concentration is 0.5E+6[/L].

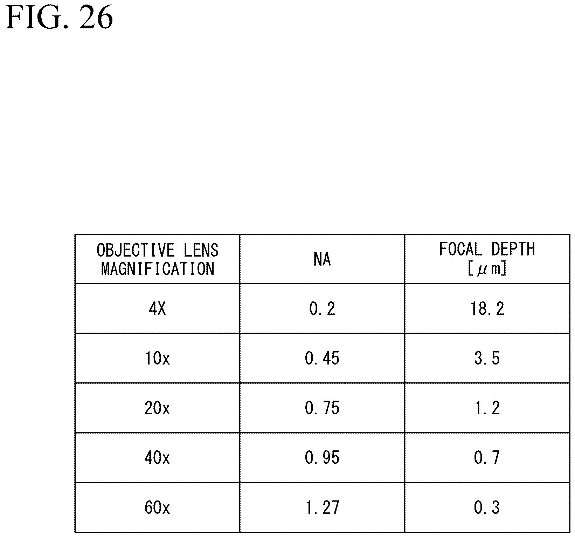

FIG. 26 is a diagram illustrating an example of a relationship between the objective lens NA and the focal depth of the objective lens.

An example in which an observation wavelength ($\lambda$) is 550 [nm] and a medium refractive index (n) is 1.33 will be described below.

The focal depth was calculated by Expression (8).

$$\text{Focal depth}=0.5\times\lambda\times n/(n-\text{sqrt}(n\,\hat{}\,2-NA\,\hat{}\,2) \quad …(8)$$

The thickness T of the accommodation unit is determined on the basis of the cell diameter D of an observation target and the in-focus range of the observation optical system.

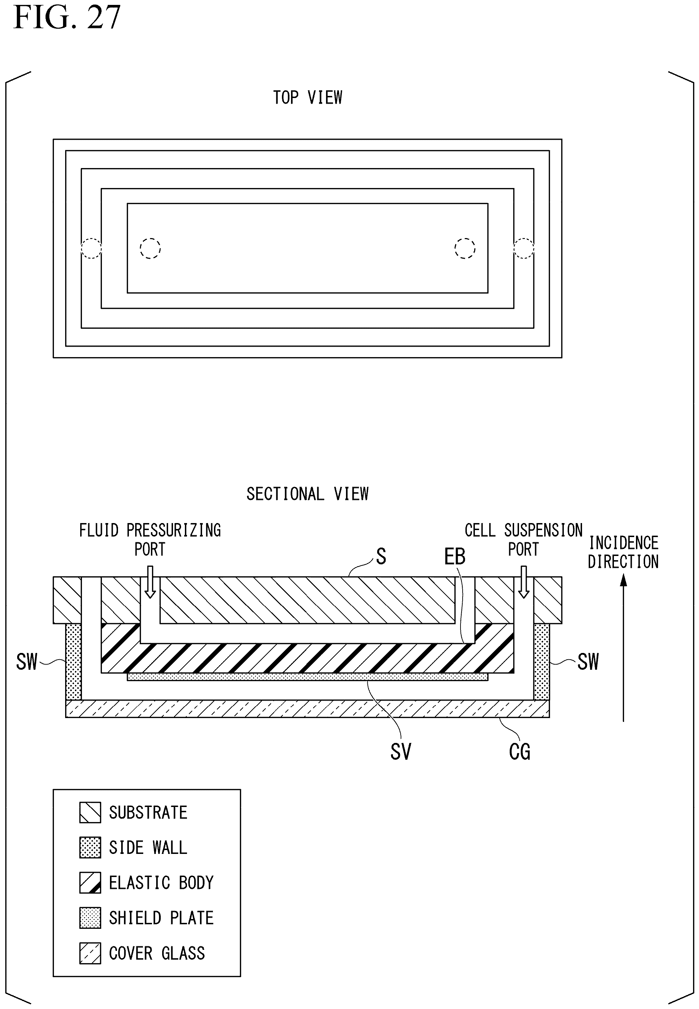

FIG. 27 is a diagram illustrating an example of an observation container. When there are different cell diameters, thin-layer containers corresponding to the cell diameters are necessary. Since cells are elastic, it is considered that there is no problem even when they are pressed to a certain extent. This is because cells may be crushed due to the pressing but can be observed. The observation container dynamically controls the thickness of the cell suspension.

The observation container includes a substrate S (a rear plate layer), an elastic body EB, a shield plate SV, a cover glass CG (a surface plate layer), and a side wall SW. The substrate 2, the elastic body EB, the shield plate SV, and the cover glass CG are sequentially stacked in a direction opposite to the incidence direction. The side wall SW is provided at an end at which the substrate 2, the elastic body EB, the shield plate SV, and the cover glass CG are sequentially stacked. The accommodation unit is formed between the shield plate SV and the cover glass CG in the incidence direction.

A cell suspension port and a fluid pressurizing port are formed in the substrate S. A cell suspension is dispensed into the container via the cell suspension port. When a fluid is dispensed via the fluid pressurizing port, the elastic body EB is expanded to change the thickness of the cell suspension. The elastic body EB is formed of an expandable material such as urethane. The thickness of the cell suspension is controlled using an amount of pressurization from the fluid pressurizing port. For example, the thickness of the cell suspension is controlled in a range of 5 $\mu$o 10 $\mu$m by controlling the amount of pressurization from the fluid pressurizing port.

FIG. 28 is a diagram illustrating an example of the observation container. The left part of FIG. 28 illustrates the observation container of FIG. 27 used as an observation container for a transmission illumination, and the right part illustrates the observation container used as an observation container for a reflection illumination.

In the observation container for a transmission illumination, the substrate S is a transparent substrate TS, and a transmission illumination TL is disposed on an incidence side of the transparent substrate TS. In the observation container for a reflection illumination, the substrate S is an opaque substrate OS.

An inner shape of an accommodation unit which is another example of the observation container will be described below.

The example of the accommodation unit of the observation container is a micro flow channel type, and the cell suspension is guided in the thin-layer container using the surface tension. Since the flow rate in the flow rate distribution is high at the center of the flow channel, cells are likely to be locally concentrated at the central part.

FIG. 29A is a diagram illustrating Example 1 of the observation container. In FIG. 29A, the upper part is a diagram illustrating the observation container when seen from the minus side of the Z axis, and the lower part is a diagram illustrating the observation container when seen in the direction perpendicular to the Z axis.

The observation container includes a cover CU and a base B. For example, the cover CU and the base B are formed of a resin. When the observation container is seen from the minus side of the Z axis, the shape of the cover CU is rectangular and has a longitudinal direction parallel to the X-axis direction and a short direction parallel to the Y-axis direction. When the observation container is seen in the direction perpendicular to the Z axis, the base B includes a recessed part serving as a flow channel of a cell suspension. For example, the recessed part has a longitudinal direction parallel to the X-axis direction, a short direction parallel to the Y-axis direction, and a height (thickness) direction parallel to the Z-axis direction.

In the cover CU, an injection port for injecting a cell suspension is provided on the minus side of the Z axis at one end in the longitudinal direction of the recessed part, and a discharge port for discharging the cell suspension is provided on the minus side of the Z axis at the other end. One or more observation spots which are positions for observing cells are formed in the flow channel. In FIG. 29A, five observation spots are illustrated. The length in the Y-axis direction of the flow channel is, for example, about 15 mm, the length in the X-axis direction is, for example, about 100 mm, and the length in the height direction is, for example, two times to three times the cell size (diameter).

In the cover CU, at least a part of which the plus side of the Z axis corresponds to the recessed part is transparent. With this configuration, it is possible to observe cells from the minus side of the Z axis. By forming a flow channel, a troublesome operation is unnecessary in comparison with the container described above with reference to FIG. 8A. Specifically, it is possible to make an operation of removing a release sheet unnecessary, to make an operation quantitatively dispensing the cell suspension, and to make sealing of the cover glass unnecessary. Since sealing of the cover glass is made to be unnecessary, it is possible to decrease a risk of mixture of bubbles.

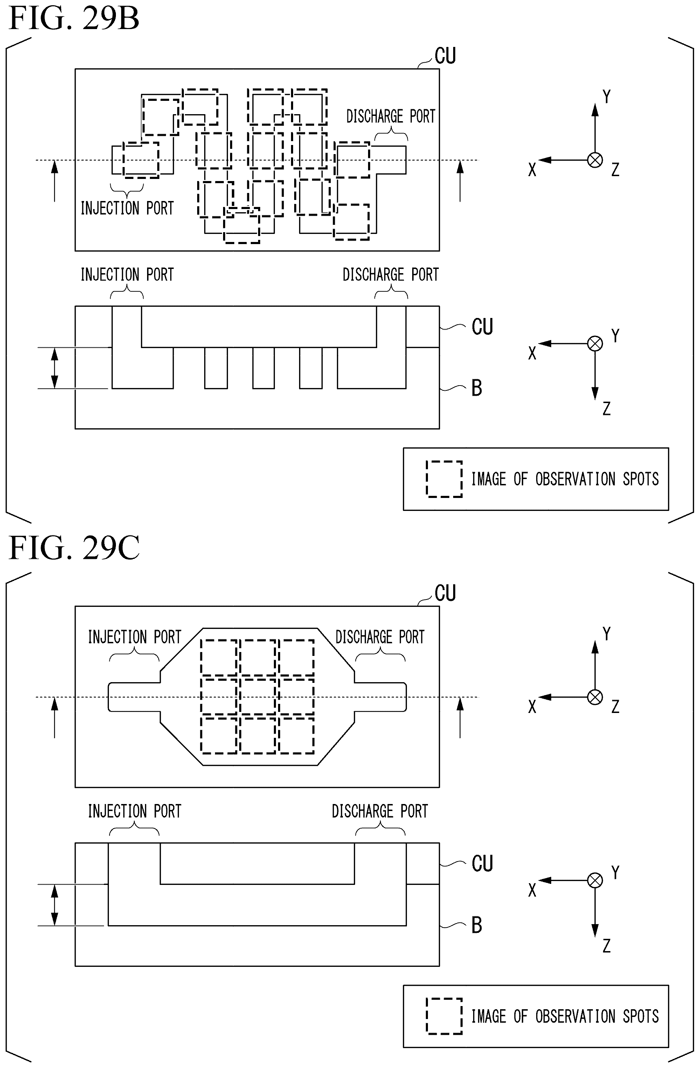

FIG. 29B is a diagram illustrating Example 2 of the observation container. In FIG. 29B, the upper part is a diagram illustrating the observation container when seen from the minus side of the Z axis, and the lower part is a diagram illustrating the observation container when seen in the direction perpendicular to the Z axis. The observation container includes a cover CU and a base B. For example, the cover CU and the base B are formed of a resin. When the observation container is seen from the minus side of the Z axis, the shape of the cover CU is rectangular and has a longitudinal direction parallel to the X-axis direction and a short direction parallel to the Y-axis direction. When the observation container is seen in the direction perpendicular to the Z axis, the base B includes a recessed part serving as a flow channel of a cell suspension. For example, the recessed part meanders in the direction parallel to the X axis and the direction parallel to the Y axis in the X-axis direction and the Y-axis direction and has a height (depth) direction parallel to the Z-axis direction.

In the cover CU, an injection port for injecting a cell suspension is provided on the minus side of the Z axis at one end of the flow channel, and a discharge port for discharging the cell suspension is provided on the minus side of the Z axis at the other end. One or more observation spots which are positions for observing cells are formed in the flow channel. In FIG. 29B, 14 observation spots are illustrated.

The length in the X-axis direction or the Y-axis direction of the flow channel is, for example, about 15 mm, the length in the height direction is, for example, two times to three times the cell size (diameter). In the cover CU, at least a part of which the plus side of the Z axis corresponds to the recessed part is transparent. With this configuration, in addition to the advantages of FIG. 29A, since a meandering flow channel can be provided, it is possible to dispose a plurality of observation spots in the observation container and to save a space of the observation container. Specifically, when the number of observation spots in FIG. 29B is the same as in FIG. 29A, a dimension in the X direction of the cover CU and the base B can be shortened by causing the flow channel to meander, and thus it is possible to save a space in the X direction. In this case, it is possible to achieve a decrease in device size in the X direction and to decrease an amount of liquid by shortening the length of the flow channel in the X direction.

Since the flow channel meanders, there is a likelihood that the dimension in the Y direction will increase. In addition, when the dimensions in the X direction of the cover CU and the base B are the same as in FIG. 29A, it is possible to increase the number of observation spots.

FIG. 29C is a diagram illustrating Example 3 of the observation container. In FIG. 29C, the upper part is a diagram illustrating the observation container when seen from the minus side of the Z axis, and the lower part is a diagram illustrating the observation container when seen in the direction perpendicular to the Z axis. The observation container includes a cover CU and a base B. For example, the cover CU and the base B are formed of a resin. When the observation container is seen from the minus side of the Z axis, the shape of the cover CU is rectangular and has a longitudinal direction parallel to the X-axis direction and a short direction parallel to the Y-axis direction. When the observation container is seen in the direction perpendicular to the Z axis, the base B includes a recessed part serving as a flow channel of a cell suspension. For example, the recessed part has a longitudinal direction parallel to the X-axis direction, a short direction parallel to the Y-axis direction, and a height (depth) direction parallel to the Z-axis direction. A part of the flow channel in the Y-axis direction is expanded.

In the cover CU, an injection port for injecting a cell suspension is provided on the minus side of the Z axis at one end in the longitudinal direction of the recessed part, and a discharge port for discharging the cell suspension is provided on the minus side of the Z axis at the other end. One or more observation spots which are positions for observing cells are formed in the flow channel. In FIG. 29C, nine observation spots are illustrated.

The length in the Y-axis direction of a part of the flow channel which is not expanded is, for example, about 15 mm, and the length in the Y-axis direction of the expanded part of the flow channel is about 45 mm. The length in the X-axis direction of the flow channel is, for example, about 100 mm, and the length in the height direction is, for example, two times to three times the cell size (diameter). In the cover CU, at least a part of which the plus side of the Z axis corresponds to the recessed part is transparent. With this configuration, it is possible to observe cells from the minus side of the Z axis. With this configuration, in addition to the advantages of FIG. 29A, since an expanded flow channel can be provided, it is possible to save a space of the observation container. Specifically, when the number of observation spots in FIG. 29C is the same as in FIG. 29A, the dimensions in the X direction of the cover CU and the base B can be shortened by employing the expanded flow channel shape, and thus it is possible to save a space in the X direction. In this case, it is possible to achieve a decrease in device size in the X direction and to decrease an amount of liquid by shortening the length of the flow channel in the X direction.

Since the expanded flow channel shape is employed, there is a likelihood that the dimension in the Y direction will increase. In addition, when the dimensions in the X direction of the cover CU and the base B are the same as in FIG. 29A, it is possible to increase the number of observation spots.

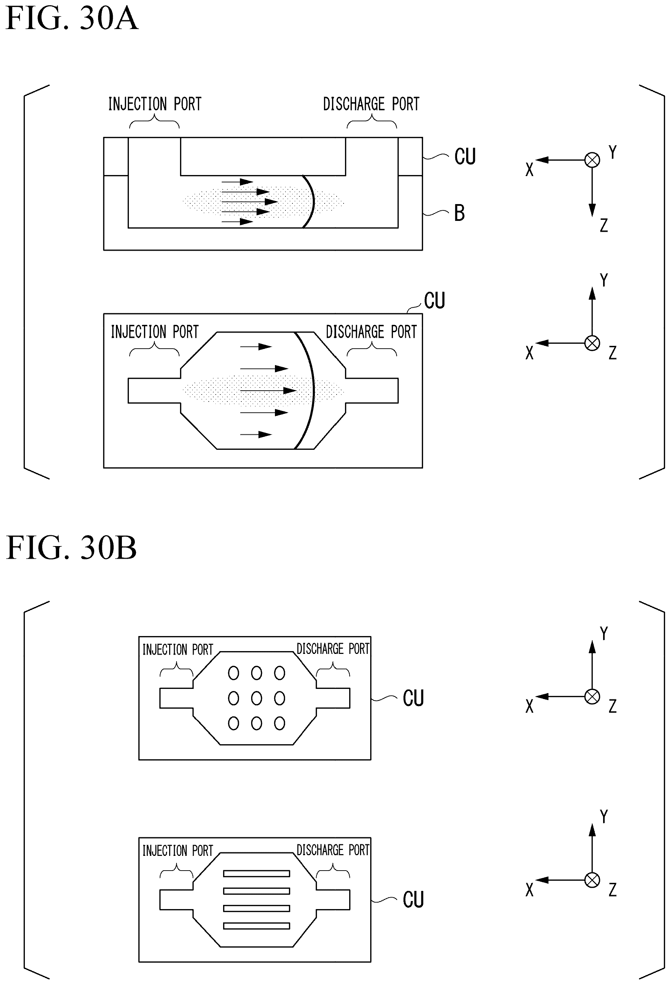

FIG. 30A is a diagram illustrating Example 4 of the observation container. In Example 4 of the observation container, the cell suspension flowing in the flow channel in FIG. 29C is made to flow as a laminar flow. It is assumed that cells are gathered at the center of the flow channel due to a flow rate distribution when the cell suspension is introduced into the flow channel via the injection port. With this configuration, it is possible to reproducibly localize the cells.

FIG. 30B is a diagram illustrating Example 5 of the observation container. FIG. 30B illustrates another example of the observation container described with reference to FIG. 29C. In the upper part of FIG. 30B, one or more columns (pillars) are arranged in the Z-axis direction in the flow channel. The pillars are formed of, for example, a resin. The pillars may be provided in the cover CU or may be provided in the base B. In this example, nine pillars are provided. In the lower part, one or more grooves are arranged in the Z-axis direction in the flow channel. The grooves are formed of, for example, a resin. In this example, four grooves are arranged. The grooves may be provided in the cover CU or may be provided in the base B. With this configuration, it is possible to control hydrophilicity and water repellency of the surface by performing reformation or surface treatment according to necessity.

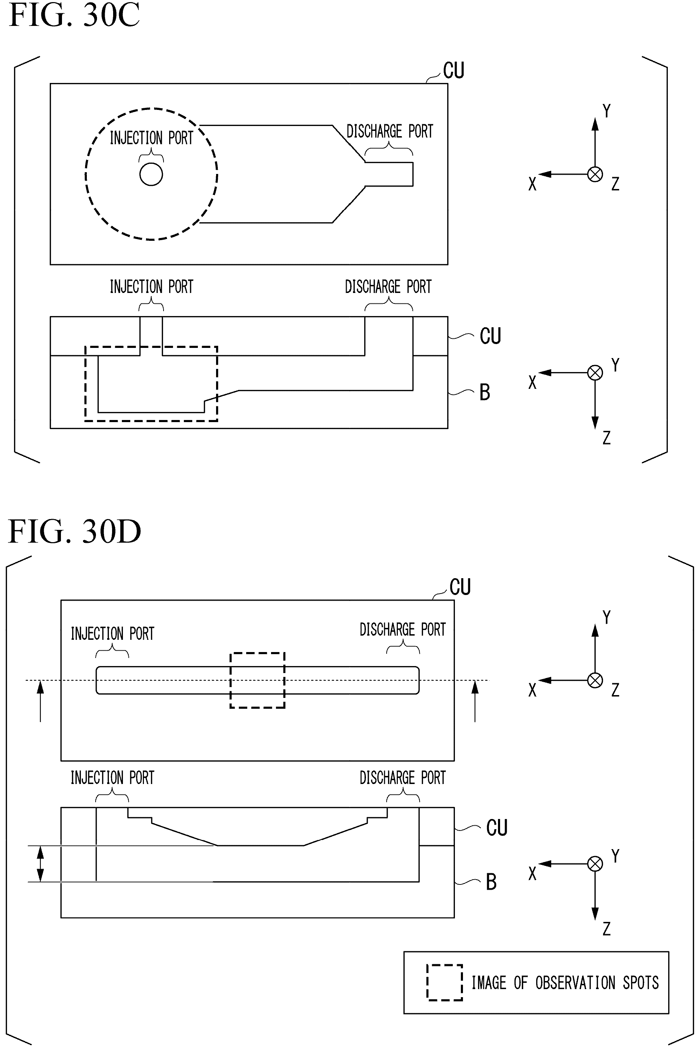

FIG. 30C is a diagram illustrating Example 6 of the observation container. In FIG. 30C, the upper part is a diagram illustrating the observation container when seen from the minus side of the Z axis, and the lower part is a diagram illustrating the observation container when seen in the direction perpendicular to the Z axis. The observation container includes a cover CU and a base B. For example, the cover CU and the base B are formed of a resin. When the observation container is seen from the minus side of the Z axis, the shape of the cover CU is rectangular and has a longitudinal direction parallel to the X-axis direction and a short direction parallel to the Y-axis direction. When the observation container is seen in the direction perpendicular to the Z axis, the base B includes a recessed part serving as a flow channel of a cell suspension. For example, the recessed part has a longitudinal direction parallel to the X-axis direction, a short direction parallel to the Y-axis direction, and a height (depth) direction parallel to the Z-axis direction. A part of the flow channel in the Y-axis direction is expanded. In the cover, an injection port for injecting a cell suspension is provided on the minus side of the Z axis at one end in the longitudinal direction of the recessed part, and a discharge port for discharging the cell suspension is provided on the minus side of the Z axis at the other end. In addition, a space is provided on the plus side of the Z axis of the injection port in the base B. With this configuration, when the cell suspension is introduced into the flow channel via the injection port, a dispersed state of cells is assumed to be easily maintained.

FIG. 30D is a diagram illustrating Example 7 of the observation container. In FIG. 30D, the upper part is a diagram illustrating the observation container when seen from the minus side of the Z axis, and the lower part is a diagram illustrating the observation container when seen in the direction perpendicular to the Z axis. The observation container includes a cover CU and a base B. For example, the cover CU and the base B are formed of a resin. When the observation container is seen from the minus side of the Z axis, the shape of the cover CU is rectangular and has a longitudinal direction parallel to the X-axis direction and a short direction parallel to the Y-axis direction. When the observation container is seen in the direction perpendicular to the Z axis, the base B includes a recessed part serving as a flow channel of a cell suspension. For example, the recessed part has a longitudinal direction parallel to the X-axis direction, a short direction parallel to the Y-axis direction, and a height (depth) direction parallel to the Z-axis direction..

In the cover CU, an injection port for injecting a cell suspension is provided on the minus side of the Z axis at one end in the longitudinal direction of the recessed part, and a discharge port for discharging the cell suspension is provided on the minus side of the Z axis at the other end. One or more observation spots which are positions for observing cells are formed in the flow channel. In FIG. 30D, one observation spot is illustrated. The thickness in the Z-axis direction of the cover CU is machined such that the height of the flow channel other than the observation spot increases. In the flow channel, the length in the Y-axis direction is, for example, about 15 mm, the length in the X-axis direction is, for example, about 100 mm, and the length in the height direction is, for example, two times to three times the cell size (diameter). In the cover CU, at least a part of which the plus side of the Z axis corresponds to the recessed part is transparent. With this configuration, since the height of the flow channel other than the observation spot can be increased, it is possible to decrease a pressure loss and to make the cel suspension to easily flow.

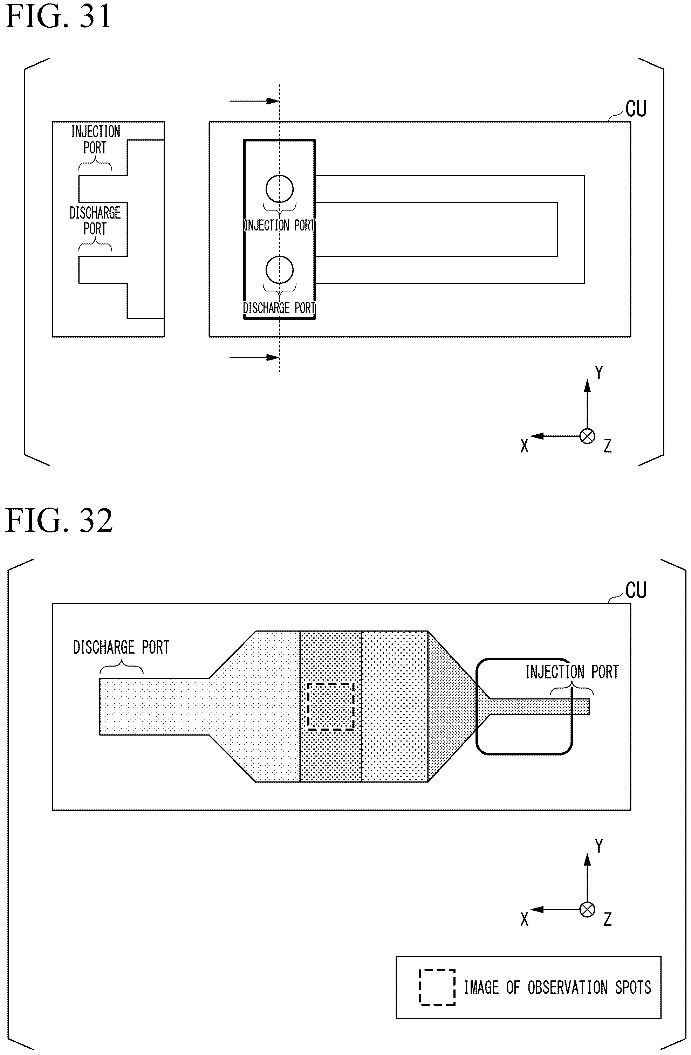

FIG. 31 is a diagram illustrating Example 8 of the observation container. FIG. 31 is a diagram illustrating the observation container when seen from the minus side of the Z axis. The observation container when seen in the direction perpendicular to the Z axis is the same as described above (FIG. 29A) and thus will not be described. In Example 7 of the observation container, an injection port and a discharge port are provided at positions which can merge. With this configuration, when a cell suspension is injected via the injection port, a position of an interface can be arranged and thus it is possible to stop a flow of the cell suspension.

FIG. 32 is a diagram illustrating Example 9 of the observation container. FIG. 32 is a diagram illustrating the observation container when seen from the minus side of the Z axis. The observation container when seen in the direction perpendicular to the Z axis is the same as described above (FIG. 29C) and thus will not be described. When the observation container is seen from the minus side of the Z axis, the shape of the cover CU is rectangular and has a longitudinal direction parallel to the X-axis direction and a short direction parallel to the Y-axis direction. The base B includes a recessed part serving as a flow channel of a cell suspension. For example, the recessed part has a longitudinal direction parallel to the X-axis direction, a short direction parallel to the Y-axis direction, and a height (depth) direction parallel to the Z-axis direction. A part of the flow channel in the Y-axis direction is expanded.

In the cover CU, an injection port for injecting the cell suspension is provided on the minus side of the Z axis at one end in the longitudinal direction of the recessed part, and a discharge port for discharging the cell suspension is provided on the minus side of the Z axis at the other end. The length in the Y-axis direction of the discharge port is shorter than the length in the Y-axis direction of the injection port. With this configuration, since a concentration distribution of cells can be locally distributed, it is possible to observe a part with a desired concentration even when cell concentrations of sample liquids are uneven.

In the cover, an injection port for injecting the cell suspension and a discharge port for discharging the cell suspension may be provided on the minus side of the Z axis in two arbitrary parts of the recessed part.

With the aforementioned observation containers, there is provided an observation container including an accommodation unit for accommodating a medium and a plurality of cells and used to acquire and observe a quantitative phase image of the plurality of cells. The accommodation unit is formed such that the thickness of the accommodation unit in the optical-axis direction of the observation optical system satisfies conditions in which the cells floating in the medium are made to stay in the in-focus range of the observation optical system for a predetermined observation time.

In the observation container, the inner surface shape of the accommodation unit is such a shape that the plurality of cells to be observed are arranged in a direction perpendicular to the optical axis of the observation optical system and in the in-focus range.

With the accommodation unit having the aforementioned configuration, it is possible to increase the number of cells staying in the in-focus range (in the focal depth) of the observation optical system.

In the observation container, the thickness of the accommodation unit in the optical-axis direction of the observation optical system is determined on the basis of a diameter of the cells to be observed. With this configuration, sine the thickness of the accommodation unit in the optical-axis direction of the observation optical system can be determined on the basis of the diameter of the cells to be observed, it is possible to increase the number of cells staying in the in-focus range of the observation optical system.

In the observation container, the thickness of the accommodation unit is determined on the basis of the diameter of the cells to be observed and the in-focus range of the observation optical system. With this configuration, since the thickness of the accommodation unit can be determined on the basis of the diameter of the cells to be observed and the in-focus range of the observation optical system, it is possible to increase the number of cells staying in the in-focus range of the observation optical system.

In the observation container, the inner surface shape of the accommodation unit includes an inner surface forming an inner space (a space of various shapes such as a column, a prism, and a flow channel) extending in the direction perpendicular to the optical-axis direction and curved to narrow or narrowing stepwise in the thickness (height) direction of the accommodation unit. With this configuration, by observing the cells in a part (region) formed to be narrow of the accommodation unit, it is possible to further increase the number of cells staying in the in-focus range of the observation optical system and to acquire a quantitative phase image of more cells.

In the observation container, the observation container has a three-layer structure, the three-layer structure includes a surface plate layer, an intermediate plate layer in which the accommodation unit is formed, and a rear plate layer, and an injection port for injecting a liquid of the medium and a discharge port for discharging the liquid of the medium are formed in the rear plate layer.

With this configuration, since a flow channel can be formed, it is possible to make a troublesome operation such as an operation of removing a release sheet, an operation of quantitatively dispensing the cell suspension with accuracy, and an operation of sealing the cover glass unnecessary.

In the observation container, a material of the observation container is a resin. With this configuration, it is possible to easily manufacture the observation container.

While embodiments of the present invention have been described above in detail with reference to the drawings, any specific configuration is not limited to the embodiments and includes design modifications without departing from the gist of the present invention.

[Reference Signs List]

1, 1a Analysis system
7 Imaging optical system
8 Stage
9 Detection unit
10 Transmissive illumination optical system
20 Objective optical system
21, 21a, 21b, 21c Objective lens
30 Relay optical system
40, 40a Analysis device
50 Control unit
51 Device control unit
52 Image creating unit
100 Microscope body
511 Optimal condition calculating unit
521 Phase restoration processing unit
522 Image constructing unit
53 Analysis unit
531 Acquisition unit
532, 532a Image analyzing unit
i, i1, i2, i3 Measuring plane
F Focal position
PSF Point intensity distribution
S Sample
Δx, Δy Sampling interval
Δz Focal interval

**Claims**

**1.** An analysis system comprising:

an acquisition unit configured to acquire a cell image including images of a plurality of cultured cells which are cultured in a medium; and
an image analyzing unit configured to analyze living states of the plurality of cultured cells included in the cell image on the basis of luminance values and cell diameters of the cultured cells included in the cell image.

2.  The analysis system according to claim 1, wherein the image analyzing unit derives a refractive index difference between the plurality of cultured cells included in the cell image and the medium on the basis of the luminance values and the cell diameters of the cultured cells and drives one or both of a survival rate of the plurality of cultured cells included in the cell image and a living cell density of living cells in the cultured cells on the basis of refractive index difference information for identifying the refractive index difference.

3.  The analysis system according to claim 2, wherein the acquisition unit acquires a quantitative phase image on the basis of light intensity distribution data of the cell image, and
    wherein the image analyzing unit derives information of the refractive index difference between the cultured cells included in the quantitative cell image and the medium on the basis of cell luminance values in the quantitative phase image and the cell diameters of the cultured cells.

4.  The analysis system according to claim 3, wherein the image analyzing unit derives the information of the refractive index difference on the basis of Expression (1):

$$\text{Phase value} = (2 \times \pi \times \Delta n \times \text{cell thickness})/\lambda \qquad \dots(1)$$

where $\lambda$ in Expression (1) is an observation wavelength, $\Delta n$ is a refractive index difference between a cell and a medium, a phase value corresponds to the cell luminance value of the quantitative phase image, and the cell thickness corresponds to the cell diameter, and
    wherein the information of the refractive index difference is acquired by dividing the cell luminance value by the cell diameter.

5.  The analysis system according to claim 2 or 3, wherein the image analyzing unit determines living cells and dead cells in the cultured cells on the basis of the information of the refractive index difference and derives the survival rate of the cultured cells using Expression (2) on the basis of a count result of the living cells and the number of observed cells:

$$\text{Survival rate } [\%] = 100 \times (\text{number of living cells})/(\text{number of observed cells})$$

$$\dots(2).$$

6.  The analysis system according to claim 2 or 3, wherein the image analyzing unit determines living cells and dead cells in the cultured cells on the basis of the information of the refractive index difference and derives a living cell density of the living cells using Expression (3) on the basis of a counting result of the living cells and a capacity of a container in an observation field of view:

Living cell density [cells/mL]=(number of living cells)/(observation field capacity)    (3).

7.  The analysis system according to claim 6, wherein the image analyzing unit calculates a specific antibody production rate of antibody forming cells which is an index at which the cultured cells produce antibodies on the basis of a concentration of an antibody product produced by the cultured cells and a living cell density of the living cells using the concentration of the antibody product as an input value.

8.  The analysis system according to claim 7, wherein the image analyzing unit calculates the antibody product of the antibody forming cells on the basis of a correlation between the information of the refractive index difference and the specific antibody production rate.

9.  The analysis system according to claim 5 or 6, wherein the image analyzing unit prepares a histogram with the refractive index difference as the horizontal axis and with a count as the vertical axis and determines the living cells and the dead cells.

10. The analysis system according to claim 8, wherein the image analyzing unit determines an analytical curve indicating a correlation between the specific antibody production rate and the information of the refractive index difference and calculates an antibody production on the basis of the determined analytical curve.

11. An observation container comprising an accommodation unit for accommodating a medium and a plurality of cells and used to acquire and observe a quantitative phase image of the plurality of cells,
wherein the accommodation unit is formed such that an inner surface shape of the accommodation unit in an optical-axis direction of an observation optical system satisfies conditions in which the cells floating in the medium are made to stay in an in-focus range of the observation optical system for a predetermined observation time.

12. The observation container according to claim 11, wherein the inner surface shape of the accommodation unit is such a shape that the plurality of cells to be observed are arranged in a direction perpendicular to the optical axis of the observation optical system and in the in-focus range.

13. The observation container according to claim 12, wherein a thickness of the accommodation unit in the optical-axis direction of the observation optical system is determined on the basis of a diameter of the cells to be observed.

14. The observation container according to claim 13, wherein the inner surface shape of the accommodation unit includes an inner surface forming an inner space extending in a direction perpendicular to the optical-axis direction and curved to narrow or narrowing stepwise in the thickness direction of the accommodation unit.

15. The observation container according to claim 12, wherein the observation container has a three-layer structure,

wherein the three-layer structure includes a surface plate layer, an intermediate plate layer in which the accommodation unit is formed, and a rear plate layer, and
wherein an injection port for injecting a liquid of the medium and a discharge port for discharging the liquid of the medium are formed in the rear plate layer.

16. The observation container according to claim 11, wherein a material of the observation container is a resin.

17. An analysis method that is performed by a computer, the analysis method comprising:

a step of acquiring a cell image including images of a plurality of cultured cells;
a step of deriving a refractive index difference between a medium in the plurality of cultured cells and the cells included in the cell image acquired in the acquiring step; and
a step of deriving one or both of a survival rate of the cultured cells included in the cell image and a living cell density of living cells in the cultured cells on the basis of refractive index difference information identifying the refractive index difference derived in the deriving step.

18. An analysis method that is performed by a computer, the analysis method comprising:

a step of acquiring a cell image including images of one or more cultured cells;
a step of deriving a refractive index difference between a medium in the one or more cultured cells and the cells included in the cell image acquired in the acquiring step; and
a step of determining that a group having a high refractive index difference out of the one or more cultured cells included in the cell image is cells with an excellent antibody production capacity out of the cultured cells on the basis of refractive index difference information for identifying the refractive index difference derived in the deriving step.

19. A program causing a computer to perform:

a step of acquiring a cell image including images of a plurality of cultured cells;
a step of deriving a refractive index difference between a medium in the plurality of cultured cells and the cells included in the cell image acquired in the acquiring step; and
a step of deriving one or both of a survival rate of the cultured cells included in the cell image and a living cell density of living cells in the cultured cells on the basis of refractive index difference information identifying the refractive index difference derived in the deriving step.

**20.** A program causing a computer to perform:

a step of acquiring a cell image including images of one or more cultured cells;
a step of deriving a refractive index difference between a medium and a cell in the one or more cultured cells included in the cell image acquired in the acquiring step; and
a step of determining that a group having a high refractive index difference out of the one or more cultured cells included in the cell image is cells with an excellent antibody production capacity out of the cultured cells on the basis of information identifying the refractive index difference derived in the deriving step.

# FIG. 1

MICROSCOPE BODY

# FIG. 2

```
                                                    ┌─ 40
┌──────────────────────────────────────────────────────┐
│                    ANALYSIS DEVICE                     │
│                                         ┌─ 50          │
│        ┌─────────────────────────────────────────────┐│
│        ┆            CONTROL UNIT                       ┆│
│    ┌─41 ┆                              ┌─51            ┆│
│  ┌──────────┐ ┆        ┌────────────────────────────┐ ┆│
│  │INPUT UNIT│ ┆        │   DEVICE CONTROL UNIT       │ ┆│
│  └──────────┘ ┆        │               ┌─511         │ ┆│
│    ┌─42       ┆        │  ┌───────────────────────┐  │ ┆│
│  ┌────────────┐┆       │  │ OPTIMAL CONDITION     │  │ ┆│
│  │DISPLAY UNIT│┆       │  │ CALCULATING UNIT      │  │ ┆│
│  └────────────┘┆       │  └───────────────────────┘  │ ┆│
│                ┆       └────────────────────────────┘ ┆│
│    ┌─43        ┆                       ┌─52           ┆│
│  ┌──────────────┐┆     ┌────────────────────────────┐ ┆│
│  │COMMUNICATION │┆     │   IMAGE CREATING UNIT       │ ┆│
│  │    UNIT      │┆     │               ┌─521         │ ┆│
│  └──────────────┘┆     │  ┌───────────────────────┐  │ ┆│
│                  ┆     │  │ PHASE RESTORATION     │  │ ┆│
│    ┌─44          ┆     │  │ PROCESSING UNIT       │  │ ┆│
│  ┌──────────────┐┆     │  └───────────────────────┘  │ ┆│
│  │ STORAGE UNIT │┆     │               ┌─522         │ ┆│
│  └──────────────┘┆     │  ┌───────────────────────┐  │ ┆│
│                  ┆     │  │ IMAGE CONSTRUCTING UNIT│  │ ┆│
│                  ┆     │  └───────────────────────┘  │ ┆│
│                  ┆     └────────────────────────────┘ ┆│
│                  ┆                     ┌─53           ┆│
│                  ┆     ┌────────────────────────────┐ ┆│
│                  ┆     │      ANALYSIS UNIT          │ ┆│
│                  ┆     │               ┌─531         │ ┆│
│                  ┆     │  ┌───────────────────────┐  │ ┆│
│                  ┆     │  │  ACQUISITION UNIT     │  │ ┆│
│                  ┆     │  └───────────────────────┘  │ ┆│
│                  ┆     │               ┌─532         │ ┆│
│                  ┆     │  ┌───────────────────────┐  │ ┆│
│                  ┆     │  │  IMAGE ANALYZING UNIT │  │ ┆│
│                  ┆     │  └───────────────────────┘  │ ┆│
│                  ┆     └────────────────────────────┘ ┆│
│        └─────────────────────────────────────────────┘│
└──────────────────────────────────────────────────────┘
```

EP 4 389 871 A1

# FIG. 3

PLANE WAVE    SAMPLE    WAVEFRONT    MEASUREMENT PLANE

A3

A4

W1    W2    W3    S    W4    i1

# FIG. 4

SAMPLE    DETECTION PLANE

7

IMAGING OPTICAL SYSTEM

EMISSION OF LIGHT

IMAGE (INTENSITY IMAGE)

$z-\Delta z$  $z$  $z+\Delta z$

MEASUREMENT PLANE    i2    i1    i3

Z

$I2(x,y,z-\Delta z)$    $I1(x,y,z)$    $I3(x,y,z+\Delta z)$    LIGHT INTENSITY DISTRIBUTION

$$\frac{\delta}{\delta z}I(x,y,z)$$

35

FIG. 5

FIG. 6

# FIG. 7

（A）

PSF

i

P

P

（B）

PSF

i

2P

2P

# FIG. 8A

EP 4 389 871 A1

## FIG. 8B

(1)  (2)

BLURRED REGION

IN-FOCUS RANGE

BLURRED REGION

○ FLOATING CELL

MEDIUM

OBSERVATION
LUMINOUS FLUX

## FIG. 9

BRIGHT-FIELD IMAGE

393.15x393.15 microns (1608x1608); 16-bit; 4.9MB

FIG. 10

QUANTITATIVE PHASE IMAGE

FIG. 11

QUANTITATIVE PHASE ANALYSIS RESULT

# FIG. 12

PHASE IN CELL IS LARGE          PHASE IN CELL IS MIDDLE          PHASE IN CELL IS SMALL

○ LOW-REFRACTIVE MOLECULE
(MEDIUM COMPONENT)

FIG. 13

OVERLAP IMAGE OF MASK AND
BRIGHT-FIELD IMAGE CREATED
USING THRESHOLD VALUE 1000

OVERLAP IMAGE OF MASK AND
BRIGHT-FIELD IMAGE CREATED
USING THRESHOLD VALUE 750

OVERLAP IMAGE OF MASK AND
BRIGHT-FIELD IMAGE CREATED
USING THRESHOLD VALUE 500

# FIG. 14

EP 4 389 871 A1

START

**S1-1** PLACE SAMPLE ON STAGE

**S2-1** INPUT INFORMATION ON MEASUREMENT CONDITIONS

**S3-1** ACQUIRE PARAMETERS ON THE BASIS OF INFORMATION INPUT BY USER

**S4-1** CALCULATE FOCAL INTERVAL $\Delta z$ ON THE BASIS OF ACQUIRED PARAMETERS

**S5-1** CALCULATE NUMBER OF POSITIONS AT WHICH FOCAL POINT OF OBJECTIVE LENS IS DISPOSED

**S6-1** DRIVE XY STAGE AND SET POSITION OF IN-FOCUS PLANE

**S7-1** IMAGE SAMPLE AND GENERATE LIGHT INTENSITY DISTRIBUTION DATA ON MEASUREMENT PLANE OF SAMPLE

**S8-1** PRE-PROCESS LIGHT INTENSITY DISTRIBUTION DATA ACQUIRED BY IMAGING

**S9-1** GENERATE DIFFERENTIAL COEFFICIENT DISTRIBUTION DATA OF LIGHT INTENSITY ON MEASUREMENT PLANE WITH RESPECT TO $z$ FROM PRE-PROCESSED LIGHT INTENSITY DISTRIBUTION DATA

**S10-1** GENERATE PHASE DISTRIBUTION DATA FROM DIFFERENTIAL COEFFICIENT DISTRIBUTION DATA OF LIGHT INTENSITY ON MEASUREMENT PLANE WITH RESPECT TO $z$ ON THE BASIS OF INTENSITY TRANSPORT DATA

**S11-1** GENERATE AND STORE QUANTITATIVE PHASE IMAGE OF SAMPLE ON THE BASIS OF GENERATED PHASE DISTRIBUTION DATA

END

# FIG. 15

START

↓

**S1-2**: DETERMINE ROUNDNESS THRESHOLD VALUE

↓

**S2-2**: GENERATE CELL BODY MASK BY DETERMINING AND BINARIZING QUANTITATIVE PHASE IMAGE THRESHOLD VALUE

↓

**S3-2**: SEPARATE NEIGHBORING CELL BODY MASKS

↓

**S4-2**: EXCLUDE CELL BODY MASKS PRESENT ON IMAGE EDGE

↓

**S5-2**: CALCULATE ROUNDNESS OF CELL BODY MASKS

↓

**S6-2**: EXTRACT CELL BODY MASKS OF WHICH ROUNDNESS IS EQUAL TO OR GREATER THAN ROUNDNESS THRESHOLD VALUE

↓

**S7-2**: CALCULATE CELL DIAMETER OF CELL BODY MASK

↓

**S8-2**: CAUSE CELL BODY MASKS TO OVERLAP QUANTITATIVE PHASE IMAGE AND CALCULATE MAXIMUM LUMINANCE VALUE IN CELL BODY MASK

↓

**S9-2**: CALCULATE MAXIMUM LUMINANCE VALUE/CELL DIAMETER AND CALCULATE REFRACTIVE INDEX DIFFERENCE $\Delta n$ BETWEEN CELL AND MEDIUM

↓

**S10-2**: CREATE HISTOGRAM OF $\Delta n$

↓

END

EP 4 389 871 A1

FIG. 16

```
           ┌─────────────┐
           │    START    │
           └──────┬──────┘
                  │
                  ▼
   ┌──────────────────────────────┐
   │    EXTRACT CULTURED CELLS     │─── S1-3
   └──────────────┬───────────────┘
                  │
                  ▼
   ┌──────────────────────────────┐
   │   PERFORM FLOW OF CREATING    │─── S2-3
   │   QUANTITATIVE PHASE IMAGE    │
   └──────────────┬───────────────┘
                  │
                  ▼
   ┌──────────────────────────────────┐
   │ PERFORM FLOW OF CALCULATING       │─── S3-3
   │ REFRACTIVE INDEX DIFFERENCE (Δn)  │
   │ BETWEEN CELL AND MEDIUM           │
   └──────────────┬───────────────────┘
                  │
                  ▼
   ┌──────────────────────────────┐
   │ DETERMINE LIVING-DEAD         │─── S4-3
   │ DETERMINATION THRESHOLD VALUE │
   └──────────────┬───────────────┘
                  │
                  ▼
   ┌──────────────────────────────┐
   │  CALCULATE SURVIVAL RATE AND  │─── S5-3
   │  LIVING CELL DENSITY          │
   └──────────────┬───────────────┘
                  │
                  ▼
   ┌──────────────────────────────┐
   │         OUTPUT RESULT         │─── S6-3
   └──────────────┬───────────────┘
                  │
                  ▼
           ┌─────────────┐
           │     END     │
           └─────────────┘
```

FIG. 17

FIG. 18

```
                                                    ┌─40a
┌──────────────────────────────────────────────────────┐
│                    ANALYSIS DEVICE                     │
│                                         ┌─50a          │
│              ┌ ─ ─ ─ ─ ─ ─ ─ ─ ─ ─ ─ ─ ─ ─ ─ ─ ─ ─ ┐  │
│                           CONTROL UNIT                 │
│              │                                      │  │
│   ┌─41          ┌─51                                   │
│ ┌──────────┐ │ ┌──────────────────────────────────┐│  │
│ │INPUT UNIT│   │      DEVICE CONTROL UNIT          │   │
│ └──────────┘ │ │               ┌─511               ││  │
│                │   ┌───────────────────────────┐   │   │
│   ┌─42       │ │   │  OPTIMAL CONDITION        │   ││  │
│ ┌───────────┐  │   │  CALCULATING UNIT         │   │   │
│ │DISPLAY UNIT││ │   └───────────────────────────┘   ││  │
│ └───────────┘  └──────────────────────────────────┘   │
│              │                         ┌─52         │  │
│   ┌─43         ┌──────────────────────────────────┐   │
│ ┌────────────┐│ │      IMAGE CREATING UNIT         ││  │
│ │COMMUNICATION│ │               ┌─521               │   │
│ │   UNIT     ││ │   ┌───────────────────────────┐   ││  │
│ └────────────┘ │   │  PHASE RESTORATION        │   │   │
│              │ │   │  PROCESSING UNIT          │   ││  │
│   ┌─44         │   └───────────────────────────┘   │   │
│ ┌───────────┐│ │               ┌─522               ││  │
│ │STORAGE UNIT│ │   ┌───────────────────────────┐   │   │
│ └───────────┘│ │   │  IMAGE CONSTRUCTING UNIT  │   ││  │
│                │   └───────────────────────────┘   │   │
│              │ └──────────────────────────────────┘│  │
│                                         ┌─53a         │
│              │ ┌──────────────────────────────────┐│  │
│                │        ANALYSIS UNIT              │   │
│              │ │               ┌─531               ││  │
│                │   ┌───────────────────────────┐   │   │
│              │ │   │  ACQUISITION UNIT         │   ││  │
│                │   └───────────────────────────┘   │   │
│              │ │               ┌─532a              ││  │
│                │   ┌───────────────────────────┐   │   │
│              │ │   │  IMAGE ANALYZING UNIT     │   ││  │
│                │   └───────────────────────────┘   │   │
│              │ └──────────────────────────────────┘│  │
│              └ ─ ─ ─ ─ ─ ─ ─ ─ ─ ─ ─ ─ ─ ─ ─ ─ ─ ─ ┘  │
└──────────────────────────────────────────────────────┘
```

47

# FIG. 19

EP 4 389 871 A1

## FIG. 20

SPECIFIC ANTIBODY PRODUCTION RATE
[pg/cell/day]

# FIG. 21

```
                    ┌─────────────┐
                    │    START    │
                    └─────────────┘
                           │
        ┌──────────────────┤
        │                  ▼
        │    ┌───────────────────────────────┐
        │    │  DETERMINE CULTURING PERIOD   │──── S1-4
        │    └───────────────────────────────┘
        │                  │
        │                  ▼
        │    ┌───────────────────────────────┐
        │    │     EXTRACT CULTURED CELLS    │──── S2-4
        │    └───────────────────────────────┘
        │                  │
        │                  ▼
        │    ┌──────────────────────────────────────────┐
        │    │ MEASURE ANTIBODY CONCENTRATION IN CULTURED CELLS │──── S3-4
        │    └──────────────────────────────────────────┘
        │                  │
        │                  ▼
        │    ┌───────────────────────────────┐
        │    │   PERFORM FLOW OF NONINVASIVELY │──── S4-4
        │    │   DETERMINING CELL LIFE-DEATH   │
        │    └───────────────────────────────┘
        │                  │
        │                  ▼
        │    ┌──────────────────────────────────────────┐
        │    │ CALCULATE SPECIFIC ANTIBODY PRODUCTION RATE │──── S5-4
        │    └──────────────────────────────────────────┘
        │                  │
        │                  ▼
        │    ┌───────────────────────────────┐
        │    │   CALCULATE AVERAGE FROM REFRACTIVE │──── S6-4
        │    │   INDEX DIFFERENCE (Δn)        │
        │    └───────────────────────────────┘
        │                  │
        │                  ▼
        │    ┌──────────────────────────────────────────────┐
        │    │ PLOT CORRELATION BETWEEN SPECIFIC ANTIBODY PRODUCTION │──── S7-4
        │    │ RATE AND AVERAGE OF REFRACTIVE INDEX DIFFERENCE (Δn) │
        │    └──────────────────────────────────────────────┘
        │                  │
        │                  ▼              S8-4
        │              ╱─────────────╲
        │   NO        ╱               ╲
        └────────────┤  END CULTURING PERIOD │
                      ╲               ╱
                       ╲─────────────╱
                            │ YES
                            ▼
                    ┌─────────────┐
                    │     END     │
                    └─────────────┘
```

FIG. 22

```
              ┌──────────┐
              │  START   │
              └──────────┘
                   │
                   ▼
   ┌─────────────────────────────────┐
   │     READ ANALYTICAL CURVE       │─── S1-5
   └─────────────────────────────────┘
                   │
                   ▼
   ┌─────────────────────────────────┐
   │     EXTRACT CULTURED CELLS      │─── S2-5
   └─────────────────────────────────┘
                   │
                   ▼
   ┌─────────────────────────────────┐
   │     PERFORM FLOW OF CREATING    │
   │   QUANTITATIVE PHASE IMAGE      │─── S3-5
   └─────────────────────────────────┘
                   │
                   ▼
   ┌─────────────────────────────────────┐
   │ PERFORM FLOW OF CALCULATING REFRACTIVE INDEX │
   │ DIFFERENCE (Δn) BETWEEN CELL AND MEDIUM      │─── S4-5
   └─────────────────────────────────────┘
                   │
                   ▼
   ┌─────────────────────────────────┐
   │    DERIVE AVERAGE FROM REFRACTIVE │
   │    INDEX DIFFERENCE (Δn)         │─── S5-5
   └─────────────────────────────────┘
                   │
                   ▼
   ┌─────────────────────────────────────┐
   │ CALCULATE SPECIFIC ANTIBODY PRODUCTION │
   │   ACCORDING TO ANALYTICAL CURVE      │─── S6-5
   └─────────────────────────────────────┘
                   │
                   ▼
              ┌──────────┐
              │   END    │
              └──────────┘
```

# FIG. 23

RESULT OUTPUT PARAMETER

- NUMBER OF LIVING CELLS, NUMBER OF DEAD CELLS,
  SURVIVAL RATE, LIVING CELL DENSITY
- AVERAGE CELL DIAMETER, MINIMUM CELL DIAMETER,
  MAXIMUM CELL DIAMETER
- AVERAGE ROUNDNESS, MINIMUM ROUNDNESS, MAXIMUM ROUNDNESS
- SPECIFIC ANTIBODY PRODUCTION RATE, ANTIBODY TEMPERATURE

| OBSERVATION DATE | ANTIBODY CONCENTRATION [mg/L] |
|---|---|
| 1 | 10 |
| 2 | 20 |
| 3 | 30 |
| 4 | 40 |

ANALYTICAL CURVE

EP 4 389 871 A1

FIG. 24

Warning

DETERIORATION OF CULTURED CELLS IS OBSERVED

FIG. 25

○ CELLS INSIDE OF
FOCAL DEPTH

◉ CELLS OUTSIDE OF
FOCAL DEPTH

# FIG. 26

| OBJECTIVE LENS MAGNIFICATION | NA | FOCAL DEPTH [μm] |
|---|---|---|
| 4X | 0. 2 | 18. 2 |
| 10x | 0. 45 | 3. 5 |
| 20x | 0. 75 | 1. 2 |
| 40x | 0. 95 | 0. 7 |
| 60x | 1. 27 | 0. 3 |

FIG. 27

TOP VIEW

SECTIONAL VIEW

FLUID PRESSURIZING
PORT

CELL SUSPENSION
PORT

INCIDENCE
DIRECTION

S       EB

SW

SW

SV       CG

| | SUBSTRATE |
| | SIDE WALL |
| | ELASTIC BODY |
| | SHIELD PLATE |
| | COVER GLASS |

FIG. 28

FIG. 29A

FIG. 29B

INJECTION PORT

DISCHARGE PORT

CU

Y

X ⊗ Z

INJECTION PORT

DISCHARGE PORT

CU

B

X ⊗ Y

Z

◻ (dashed) IMAGE OF OBSERVATION SPOTS

FIG. 29C

INJECTION PORT

DISCHARGE PORT

CU

Y

X ⊗ Z

INJECTION PORT

DISCHARGE PORT

CU

B

X ⊗ Y

Z

◻ (dashed) IMAGE OF OBSERVATION SPOTS

## FIG. 30A

INJECTION PORT       DISCHARGE PORT

CU

B

X ← ⊗ Y
Z ↓

Y ↑
X ← ⊗ Z

CU

INJECTION PORT       DISCHARGE PORT

## FIG. 30B

INJECTION PORT       DISCHARGE PORT

CU

Y ↑
X ← ⊗ Z

INJECTION PORT       DISCHARGE PORT

CU

Y ↑
X ← ⊗ Z

# FIG. 30C

# FIG. 30D

☐ IMAGE OF OBSERVATION SPOTS

FIG. 31

FIG. 32

## INTERNATIONAL SEARCH REPORT

| International application No. |
|---|
| **PCT/JP2022/030569** |

**A.    CLASSIFICATION OF SUBJECT MATTER**

*C12M 1/34*(2006.01)i; *C12M 1/00*(2006.01)i; *C12M 3/00*(2006.01)i; *C12Q 1/02*(2006.01)i; *G01J 9/02*(2006.01)i;
*G01N 21/45*(2006.01)i; *G01N 33/483*(2006.01)i
FI:    C12M1/34 A; C12M1/00 A; C12Q1/02; C12M3/00 Z; G01N21/45 A; G01J9/02; G01N33/483 C

According to International Patent Classification (IPC) or to both national classification and IPC

**B.    FIELDS SEARCHED**

Minimum documentation searched (classification system followed by classification symbols)

C12M1/34; C12M1/00; C12M3/00; C12Q1/02; G01J9/02; G01N21/45; G01N33/483

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| X | JP 2019-54742 A (SCREEN HOLDINGS CO., LTD.) 11 April 2019 (2019-04-11) | 1 |
|  | claims 1, 4 | |
| Y | paragraph [0005] | 1-10, 17-20 |
| Y | JP 2021-89244 A (NIKON CORP.) 10 June 2021 (2021-06-10) | 1-10, 17-20 |
|  | claim 1, paragraphs [0060]-[0062], [0065], [0071] | |
| Y | JP 2010-276585 A (OLYMPUS CORP.) 09 December 2010 (2010-12-09) | 1-10, 17-20 |
|  | claims 1, 3, paragraph [0008] | |
| X | JP 2020-190459 A (NIKON CORP.) 26 November 2020 (2020-11-26) | 11-13, 16 |
|  | claims 1, 4, fig. 6 | |
| X | JP 2020-188717 A (NIKON CORP.) 26 November 2020 (2020-11-26) | 11-13, 16 |
|  | claims 1, 6, fig. 7 | |
| A | WO 2021/033750 A1 (WASEDA UNIV.) 25 February 2021 (2021-02-25) | 1-20 |
|  | entire text, all drawings | |

☑ Further documents are listed in the continuation of Box C.        ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **03 October 2022** | **25 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
| --- |
| **PCT/JP2022/030569** |

| C. | DOCUMENTS CONSIDERED TO BE RELEVANT | |
| --- | --- | --- |
| Category* | Citation of document, with indication, where appropriate, of the relevant passages | Relevant to claim No. |
| A | WO 2016/063364 A1 (HITACHI HIGH-TECHNOLOGIES CORP.) 28 April 2016 (2016-04-28)<br>entire text, all drawings | 1-20 |
| A | WO 2017/115863 A1 (TOPPAN PRINTING CO., LTD.) 06 July 2017 (2017-07-06)<br>entire text, all drawings | 1-20 |

Form PCT/ISA/210 (second sheet) (January 2015)

## INTERNATIONAL SEARCH REPORT

| International application No. |
|---|
| **PCT/JP2022/030569** |

**Box No. III    Observations where unity of invention is lacking (Continuation of item 3 of first sheet)**

This International Searching Authority found multiple inventions in this international application, as follows:

(Invention 1) Claims 1-10 and 17-20
    Claim 1 lacks novelty in light of document 1, and thus does not have a special technical feature. However, claims 2-10, which are dependent on claim 1, have the special technical feature of "deriving differences in refractive index between more than one cultured cell in a cell image and a medium", and claims 17-20 also share the technical feature with claims 2-10. Accordingly, claims 1-10 and 17-20 are classified as invention 1.

(Invention 2) Claims 11-16
    Claims 11-16 cannot be said to share a same or corresponding technical feature with claims 2-10 classified as invention 1.
    Furthermore, claims 11-16 are not substantially identical to or similarly closely related to any of the claims classified as invention 1.
    Accordingly, claims 11-16 cannot be classified as invention 1.
    Claims 11-16 are therefore classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2022/030569**

| Patent document cited in search report | | | Publication date (day/month/year) | Patent family member(s) | | | Publication date (day/month/year) |
|---|---|---|---|---|---|---|---|
| JP | 2019-54742 | A | 11 April 2019 | (Family: none) | | | |
| JP | 2021-89244 | A | 10 June 2021 | (Family: none) | | | |
| JP | 2010-276585 | A | 09 December 2010 | (Family: none) | | | |
| JP | 2020-190459 | A | 26 November 2020 | (Family: none) | | | |
| JP | 2020-188717 | A | 26 November 2020 | (Family: none) | | | |
| WO | 2021/033750 | A1 | 25 February 2021 | (Family: none) | | | |
| WO | 2016/063364 | A1 | 28 April 2016 | US<br>entire text, all drawings<br>CN | 2017/0306287<br><br>107075437 | A1<br><br>A | |
| WO | 2017/115863 | A1 | 06 July 2017 | US<br>entire text, all drawings | 2018/0299380 | A1 | |

Form PCT/ISA/210 (patent family annex) (January 2015)

**REFERENCES CITED IN THE DESCRIPTION**

**Patent documents cited in the description**

- JP 2021132304 A
- JP 6039570 B

- JP 2007148159 A